# EUROPEAN PATENT SPECIFICATION

(11) **EP 4 511 794 B1**
(45) Date of publication and mention of the grant of the patent: **04.11.2026**
(21) Application number: 23721348.3
(22) Date of filing: 20.04.2023
(51) Int. Cl.: G06T 7/00, G06T 7/174, G06T 7/11

(54) **DELINEATION OF ONE OR MORE PARTS OF A BODY WITHIN A DIFFUSION WEIGHTED MRI IMAGE**
ABGRENZUNG EINES ODER MEHRERER TEILE EINES KÖRPERS IN EINEM DIFFUSIONSGEWICHTETEN MRT-BILD
DÉLIMITATION D'UNE OU PLUSIEURS PARTIES D'UN CORPS DANS UNE IMAGE D'IRM DE DIFFUSION

(30) Priority: 20.04.2022 GB 202205748; 04.01.2023 GB 202300101
(43) Date of publication of application: 26.02.2025
(73) Proprietor: The Institute of Cancer Research: Royal Cancer Hospital, London, Greater London SW7 3RP (GB); Cancer Research Technology Ltd., London Greater London E20 1JQ (GB)
(72) Inventor: CANDITO, Antonio, London Greater London SW7 3RP (GB); BLACKLEDGE, Matthew, London Greater London SW7 3RP (GB); HOLBREY, Richard Paul, London Greater London SW7 3RP (GB)
(74) Representative: Mewburn Ellis LLP
(86) International application number: PCT/EP2023/060309
(87) International publication number: WO 2023/203144

(56) References cited:
- US-A1- 2018 330 495
- CERANKA JAKUB ET AL: "Multi-atlas segmentation of the skeleton from whole-body MRI-Impact of iterative background masking", MAGNETIC RESONANCE IN MEDICINE, vol. 83, no. 5, 23 October 2019 (2019-10-23), US, pages 1851 - 1862, XP093066398, ISSN: 0740-3194, Retrieved from the Internet <URL:https://onlinelibrary.wiley.com/doi/full-xml/10.1002/mrm.28042> DOI: 10.1002/mrm.28042
- HADRIEN BERTRAND ET AL: "Classification of MRI data using Deep Learning and Gaussian Process-based Model Selection", ARXIV.ORG, CORNELL UNIVERSITY LIBRARY, 201 OLIN LIBRARY CORNELL UNIVERSITY ITHACA, NY 14853, 16 January 2017 (2017-01-16), XP080749415, DOI: 10.1109/ISBI.2017.7950626

## Description

### Field of the Invention

The present invention relates to a method of delineating one or more parts of a body within a diffusion weighted MRI 3D patient image.

### Background

Whole-Body Diffusion Weighted MRI (WBDWI) is a contemporary technique for staging and non-invasive assessment of treatment response to systemic therapies in patients with metastatic bone disease primarily from advanced prostate and breast cancers [1], [2]. Furthermore, it is now also suggested as first-line imaging for evaluation of Multiple Myeloma (MM) [3]-[7]. Whilst computed X-ray tomography (CT) and radionuclide bone scintigraphy (BS) are also used in clinical practice for management of these disease types [8], they demonstrate poorer sensitivity for disease detection and may afflict significant delays when assessing disease progression following treatment [9].

WBDWI also assists clinicians in evaluating the metastatic visceral and nodal disease in patients with APC and extramedullary lesions in patients with MM [38]. Visceral metastases are defined as soft tissue lesions (i.e. liver). Lymph node and visceral metastases might occur in approximately 30% of patients with APC [39]. Extramedullary lesions in patients with MM might occur adjacent to the bone (with or without connection) or extend to the soft tissue. These lesions might occur at the time of diagnosis or during treatment for approximately 4-16% and 6-20% of MM patients, respectively [40]. Importantly, WBDWI offers quantification of two imaging biomarkers for assessment of disease response following treatment: (i) the Total Diffusion Volume (TDV), reflecting the estimated tumour burden within the skeleton in millilitres, and (ii) the Apparent Diffusion Coefficient (ADC), a surrogate imaging biomarker of tumour cellularity [10]. Previous studies have demonstrated that an increase in ADC and/or decrease of TDV might suggest that the patient is benefitting from treatment [11], [12]. The technique is generally well tolerated by patients and facilitates long-term monitoring without radiation burden [13] [14]. Unfortunately, to benefit from these biomarkers radiologists are required to manually delineate regions of disease throughout the skeleton (requiring 1-2 hours depending on the volume of disease), or resort to the use of semi-automated methodologies [15], [16], which may lead to inter-observer variability in results [17]. Therefore, fully automatic segmentation of disease from WBDWI is desirable.

Atlas-based segmentation has previously been used to automatically delineate healthy tissues and subsequent tumour regions in imaging studies. By performing spatial co-registration between an unsegmented "target" image and a number of manually annotated "atlas" images with manually defined regions of interest (ROls), it is possible to transfer these ROls onto the target data [18], [19]. Several label fusion methodologies may be employed to merge results from the atlas images, including majority voting of segmentation labels or weighted approaches that incorporate the estimated quality of spatial alignment between images (e.g., pixelwise mean-square-error for images of the same modality). However, a key feature of these approaches is that the success of segmentation is significantly dependent on the success of the initial spatial alignment step.

In previous work, Almeida et al. [20] proposed an atlas-based registration for segmentation of the full skeleton and soft tissues that appear hyperintense on WBDWI. Registration between high b-value (800 or 1000 s/mm²) atlases and target images was performed, which facilitated development of a semi-automatic tool for segmenting tumour burden in patients with MM on WBDWI (mean dice score between lesions delineated by radiologists and proposed semi-automatic tool was 0.32). Arabi et al. [21] investigated the accuracy of skeleton segmentation from whole-body MR images using a number of atlas-based registration methods; skeleton segmentation was performed on 23 whole-body CT/MR image pairs using a leave-one-out cross (LOOCV) validation procedure. The average dice score between manual and derived skeleton segmentation from two different label fusion methods was 0.65 and 0.81, respectively. Lavdas et al. [22] developed an atlas segmentation approach for delineating multiple organs on T2-weighted MR-images of 51 healthy volunteers. The average dice score between manual and derived segmentation of pelvis and spine on test data was 0.79 and 0.87, respectively. Finally, Ceranka et al. [23] developed a multi-atlas registration algorithm to derive the skeleton on T1-weighted MR-images. The algorithm showed a dice score between manual and derived skeleton segmentation on average above 0.8, without considering shoulders and ribcage in 10 patients. However, the computational time of the multi-atlas registration step was 180 minutes and a further registration between the T1-weighted images and diffusion sequence was required to detect bone disease.

Despite these recent advances, automated delineation of disease from WBDWI remains a challenge. Anatomical variability between patients, complex skeletal structures caused by pathologies including scoliosis, presence of imaging artifacts due low signal-to-noise ratio (SNR) or magnetic susceptibility effects, and differences in patient pose during scanning are all possible contributors to the poor performance of inter-patient spatial alignment of WBDWI studies. Ideally, the patient datasets selected for definition of the atlas images should be representative of the target patient population for automatic segmentation.

US 2018/330495 A1 proposes an image enhancement system for bone disease evaluation.

HADRIEN BERTRAND ET AL: "Classification of MRI data using Deep Learning and Gaussian Process-based Model Selection", ARXIV.ORG, CORNELL UNIVERSITY LIBRARY, 201 OLIN LIBRARY CORNELL UNIVERSITY ITHACA, NY 14853, 16 January 2017 (2017-01-16), XP080749415, DOI: 10.1109/ISBI.2017.7950626 discusses classification of MRI data using deep learning and Gaussian process-based model selection.

The present invention has been devised in light of the above considerations.

### Summary of the Invention

The present invention improves the registration process in atlas-based segmentation of Diffusion Weighted MRI (DWI) using transfer learning, thereby helping to improve the robustness of the resulting segmentation.

In a first aspect, the present disclosure provides a computer-implemented method of delineating one or more parts of a body within a diffusion weighted MRI (preferably WBDWI) 3D patient image of a human or animal body, the method including:
providing the diffusion weighted MRI 3D patient image of the human or animal body, the patient image being formed of plural slices stacked along a direction of the body;
analysing the patient image to identify different contiguous anatomical regions of the body, the regions being distributed along said direction such that each slice of the patient image is allocated to a respective region;
providing an atlas of diffusion weighted MRI 3D ground truth images of plural other corresponding bodies containing the regions, each ground truth image being formed of plural slices stacked along a corresponding direction of the respective body, the different regions of the body being pre-identified for each ground truth image such that each slice of that ground truth image is allocated to a respective skeletal region, and one or more parts of the body of each ground truth image being pre-delineated;
registering each ground truth image to the patient image by: translating and stretching each ground truth image in its corresponding direction to align the identified regions of that ground truth image with the corresponding identified regions of the patient image;
identifying a transformation of each registered ground truth image that matches the pre-delineated parts of the body of that registered ground truth image to the corresponding parts of the body of the patient image by minimising a cost function; and
segmenting the patient image by obtaining a probability image for the corresponding parts of the body of the patient image, wherein the probability image combines the pre-delineated parts of the bodies of the ground truth images transformed according to their respective non-linear deformable transformations and weighted according to their respective cost-functions.

The diffusion weighted MRI 3D patient image of a human or animal body may contain a skeleton of said body, the patient image being analysed to identify different skeletal regions of the body (i.e. the skeletal regions being the contiguous anatomical regions). Thus conveniently, in this case, the computer-implemented method includes:
providing a diffusion weighted MRI 3D patient image of the human or animal body containing a skeleton, the patient image being formed of plural slices stacked along a direction of the body;
analysing the patient image to identify different skeletal regions of the body, the skeletal regions being distributed along said direction such that each slice of the patient image is allocated to a respective skeletal region;
providing an atlas of diffusion weighted MRI 3D ground truth images of plural other corresponding bodies containing skeletons, each ground truth image being formed of plural slices stacked along a corresponding direction of the respective body, the different skeletal regions of the body being pre-identified for each ground truth image such that each slice of that ground truth image is allocated to a respective skeletal region, and one or more parts of the body of each ground truth image being pre-delineated;
registering each ground truth image to the patient image by: translating and stretching each ground truth image in its corresponding direction to align the identified skeletal regions of that ground truth image with the corresponding identified skeletal regions of the patient image;
identifying a transformation of each registered ground truth image that matches the pre-delineated parts of the body of that registered ground truth image to the corresponding parts of the body of the patient image by minimising a cost function; and
segmenting the patient image by obtaining a probability image for the corresponding parts of the body of the patient image, wherein the probability image combines the pre-delineated parts of the bodies of the ground truth images transformed according to their respective non-linear deformable transformations and weighted according to their respective cost-functions.

Additionally or alternatively, the pre-delineated parts of the body may include the skeleton and/or one or more soft tissues.

For example, when the pre-delineated parts of the body are just the skeleton, the probability image is a skeleton probability image. In this case, the computer-implemented method includes:
providing a diffusion weighted MRI 3D patient image of the human or animal body containing a skeleton, the patient image being formed of plural slices stacked along a direction of the body;
analysing the patient image to identify different contiguous anatomical regions of the body, the regions being distributed along said direction such that each slice of the patient image is allocated to a respective region;
providing an atlas of diffusion weighted MRI 3D ground truth images of plural other corresponding bodies containing skeletons, each ground truth image being formed of plural slices stacked along a corresponding direction of the respective body, the different regions of the body being pre-identified for each ground truth image such that each slice of that ground truth image is allocated to a respective region, and the skeleton of each ground truth image being pre-delineated;
registering each ground truth image to the patient image by: translating and stretching each ground truth image in its corresponding direction to align the identified regions of that ground truth image with the corresponding identified regions of the patient image;
identifying a transformation of each registered ground truth image that matches the skeleton of that registered ground truth image to the skeleton of the patient image by minimising a cost function; and
skeletally segmenting the patient image by obtaining a skeleton probability image for the patient image that combines the pre-delineated skeletons of the ground truth images transformed according to their respective non-linear deformable transformations and weighted according to their respective cost-functions.

However, when the pre-delineated parts of the body include soft tissues as well as the skeleton, then the probability image is not just a skeleton probability image, but an image that also includes those tissues.

Indeed, in principle, the pre-delineated parts of the body can be just soft tissues, such that the probability image is a soft tissue image that does not include the skeleton, even if the skeleton is used for registering each ground truth image to the patient image.

Whether the pre-delineated parts of the body include the skeleton, one or more soft tissues or both, advantageously, by registering each ground truth image to the patient image before identifying a transformation of each registered ground truth image that matches the pre-delineated parts of the body of that registered ground truth image to the corresponding parts of the body of the patient image, the process of matching the pre-delineated parts of the body of the registered ground truth image to the corresponding parts of the body of the patient image can be significantly facilitated such that the identified transformation that provides the match is much more likely to be accurate and useful than if this registration is not performed. The task of matching the parts of the body can be viewed as a high dimensional global optimisation problem, and performance of the pre-registration helps to focus that the optimisation by reducing initial misalignments.

Moreover, by virtue of being computer-implemented, the method is automatable and thus repeatable, reproducible, consistent and fast. It provides results that are comparable with those that can be provided by expert, manual skeletal and/or soft tissue segmentation.

The analysing of the patient image to identify different regions (e.g. skeletal regions) of the body may conveniently be performed using a machine learning classifier, such as a neural network, for example a convolutional neural network such as a VGG16 model. Such a network can be trained on a dataset of images with the different regions previously identified by an expert.

The machine learning classifier may have a two or more channel input. This allows the identification of different regions of the body to be based on multiple inputs which in combination make up the overall diffusion weighted MRI 3D patient image. For example, the inputs may be an apparent diffusion coefficient map, and a corresponding signal intensity image obtained at a given b-value, such as a b = 0 s/mm² (S0) image.

The direction of the body of the patient image, and the corresponding directions of the ground truth images may typically be craniocaudal directions. The plural slices of the patient image and the plural slices of the ground truth images can then be axial slices. The identified different regions of the body can in turn include cervical, thoracic, lumbar and pelvic regions, which are consistent with performance of WBDWI.

The method may further include: analysing the patient image to delineate a reference feature extending in the direction of the body of the patient image. In this case, the ground truth images are images of plural other corresponding bodies having the reference feature. Moreover, the registering of each ground truth image to the patient image can then also include: translating in-plane each slice of each translated and stretched ground truth image to align the reference feature of the ground truth image in that slice with the reference feature of the patient image in a corresponding slice. By performing this in-plane translation, in addition to the translation and stretching of each ground truth image, initial misalignments of the registered ground truth images to the patient image can be further reduced, improving the focus of the high dimensional global optimisation performed by the subsequent matching of the parts of the body. Conveniently, the analysis of the patient image to delineate a reference feature can be performed using a neural network, for example a convolutional neural network such as a U-Net model. Such a network can be trained on a dataset of images with the reference feature previously delineated by an expert. The aligning of the reference feature of the ground truth image with the reference feature of the patient image in a corresponding slice can be performed, for example, by matching respective centroids of the reference features. When the contiguous anatomical regions are skeletal regions, by performing the in-plane translation, initial misalignments of the skeletons of the registered ground truth images to the skeleton of the patient image can be reduced. In this case, the reference feature may be a spinal cord, which is a convenient feature to use when the direction of the body of the patient image, and the corresponding directions of the ground truth images are the craniocaudal directions.

The identified transformation of each registered ground truth image that matches the pre-delineated parts of the body of that registered ground truth image to the corresponding parts of the body of the patient image may be a non-linear deformable transformation. For example, the transformation may be identified by a diffeomorphic demons registration algorithm, optionally preceded by a 3D affine transformation.

The cost function minimised by the transformation can be a mean square error cost function.

Performance of the method of the first aspect on plural diffusion weighted MRI 3D patient images allows the development of a training data set in which each of the diffusion weighted MRI 3D patient images is paired with a corresponding probability image. Advantageously, this training data can then be used to develop a deep learning model that outputs probability images in response to diffusion weighted MRI 3D patient images without reference to the atlas of diffusion weighted MRI 3D ground truth images. Accordingly, in a second aspect, the present disclosure provides a method of training a computer-implemented machine learning model, such as a neural network, which receives an input which is a diffusion weighted MRI 3D patient image of a human or animal body (e.g. containing a skeleton), and produces in response thereto an output which is a probability image corresponding to the diffusion weighted MRI 3D patient image for one or more parts of the body (e.g. a skeleton and/or one or more soft tissues), the method including:
performing the method of the first aspect for plural diffusion weighted MRI 3D patient images (e.g. containing a skeleton) to form a training data set in which each of the diffusion weighted MRI 3D patient images is paired with a corresponding probability image for one or more parts of the body; and
training the machine learning model using the training data set to minimise a cost function that, when each diffusion weighted MRI 3D patient image from the training data set is inputted into the model, measures similarity between the output of the model and the corresponding probability image.

The cost function minimised by the training can be a distribution-based loss function, such as a binary cross-entropy loss function.

In the second aspect, the machine learning model may have a two or more channel input. This allows the identification of different parts of the body to be based on multiple inputs which in combination make up the overall diffusion weighted MRI 3D patient image. For example, the inputs may be an apparent diffusion coefficient map, and a corresponding signal intensity image obtained at a given b-value, such as a b = 0 s/mm² (S0) image.

In the second aspect, the machine learning model may be a supervised deep learning segmentation model.

More generally, in either of the preceding aspects, the diffusion weighted MRI 3D patient image of the human or animal body may be a single map or image or may combine plural corresponding maps or images. For example, the image may be one of, or may combine both of, an apparent diffusion coefficient map, and a corresponding signal intensity image obtained at a given b-value, such as a b = 0 s/mm² (S0) image.

In a third aspect, the present disclosure provides a computer-implemented procedure for identifying possible regions of disease in a human or animal body containing a skeleton, the procedure including: performing the method of the first aspect; and using the probability image from the method of the first aspect to identify possible regions of disease. For example, the probability image may be used by combining the probability image with other information derived from the diffusion weighted MRI 3D patient image of the human or animal body, such as apparent diffusion coefficient information or high b-value (e.g. b = 900 s/mm² or greater) image signal information. This can then be used to determine a value for TDV, reflecting the estimated tumour burden in millilitres.

The method of the first aspect and the procedure of the third aspect are computer-implemented. Accordingly, further aspects of the present disclosure provide: a computer program comprising code which, when the code is executed on a computer, causes the computer to perform the method of the first aspect; a computer readable medium storing a computer program comprising code which, when the code is executed on a computer, causes the computer to perform the method of the first aspect; and a computer system programmed to perform the method of the first aspect. Similarly, further aspects of the present disclosure provide: a computer program comprising code which, when the code is executed on a computer, causes the computer to perform the procedure of the third aspect; a computer readable medium storing a computer program comprising code which, when the code is executed on a computer, causes the computer to perform the procedure of the third aspect; and a computer system programmed to perform the procedure of the third aspect.

In a further aspect, the present disclosure provides an imaging system for performing diffusion-weighted MRI, the system including: a magnetic resonance imaging scanner for acquiring a diffusion-weighted MRI 3D patient image of a human or animal body; and the computer system programmed to perform the method of the first aspect, or the procedure of the third aspect, the computer system being configured to communicate with the scanner such that the computer system is provided with the acquired image.

The invention includes the combination of the aspects and preferred or optional features described except where such a combination is clearly impermissible or expressly avoided.

The term "computer readable medium" may represent one or more devices for storing data, including read only memory (ROM), random access memory (RAM), magnetic RAM, core memory, magnetic disk storage mediums, optical storage mediums, flash memory devices and/or other machine readable mediums for storing information. The term "computer-readable medium" includes, but is not limited to portable or fixed storage devices, optical storage devices, wireless channels and various other mediums capable of storing, containing or carrying instruction(s) and/or data.

Furthermore, embodiments may be implemented by hardware, software, firmware, middleware, microcode, hardware description languages, or any combination thereof. When implemented in software, firmware, middleware or microcode, the program code or code segments to perform the necessary tasks may be stored in a computer readable medium. One or more processors may perform the necessary tasks. A code segment may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

### Summary of the Figures

Embodiments and experiments illustrating the principles of the invention will now be discussed with reference to the accompanying figures in which:

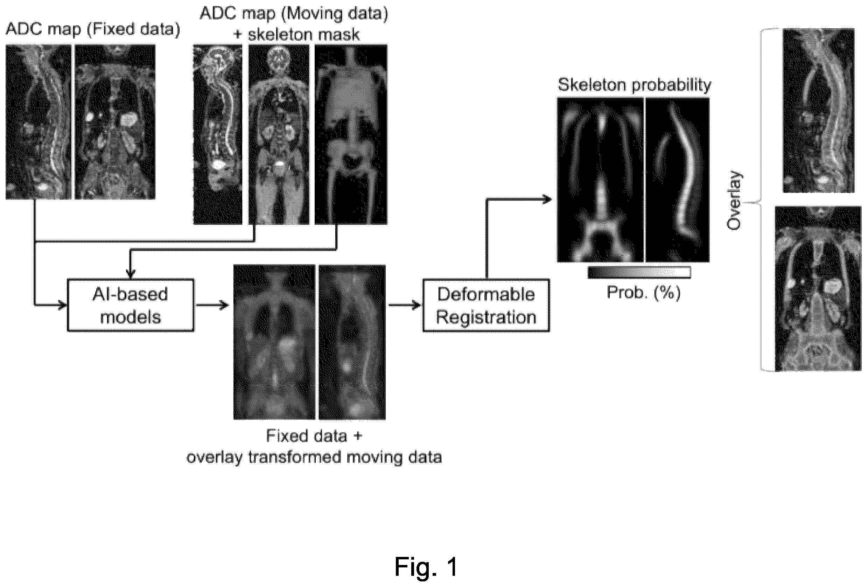

**Figure 1****.** Schematic representation of WBDWI atlas-based registration. Multiple non-linear deformable registrations are performed between the target (ADC map - 'fixed data') and atlas image (ADC map - 'moving image'). The derived transforms are applied to the pre-contoured atlas mask. The procedure is repeated for all patients in the atlas cohort. Transformed masks are combined using a fusion label technique to derive the skeleton probability image. A colour map of the skeleton probability image is superimposed on coronal and sagittal view of ADC maps. The skeleton probability image shows lower uncertainty (values close '1') for voxels that belong to the skeleton image.

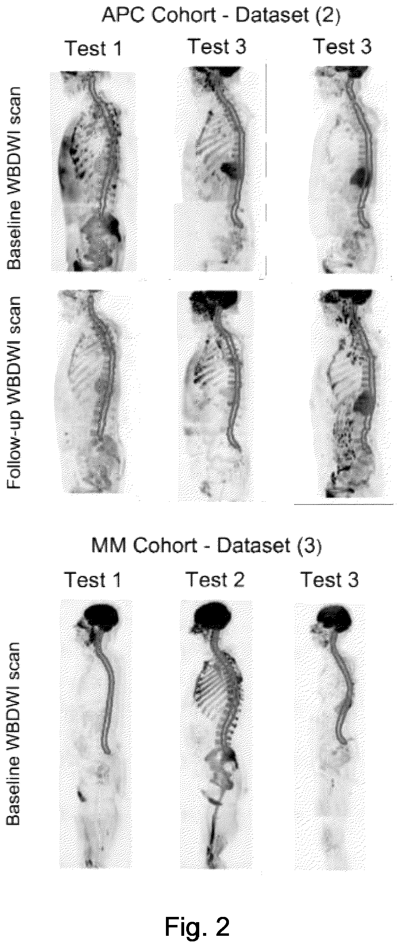

**Figure 2****.** Coronal and sagittal view of Maximum-Intensity-Projection (MIP) of b = 900 s/mm² image and superimposed derived spinal cord segmentation for six test datasets: three patients with confirmed MM from **Dataset (2)** and three patients with confirmed APC from **Dataset (3).** The Al-based model predicts with low uncertainty the position of the spinal cord for all test datasets.

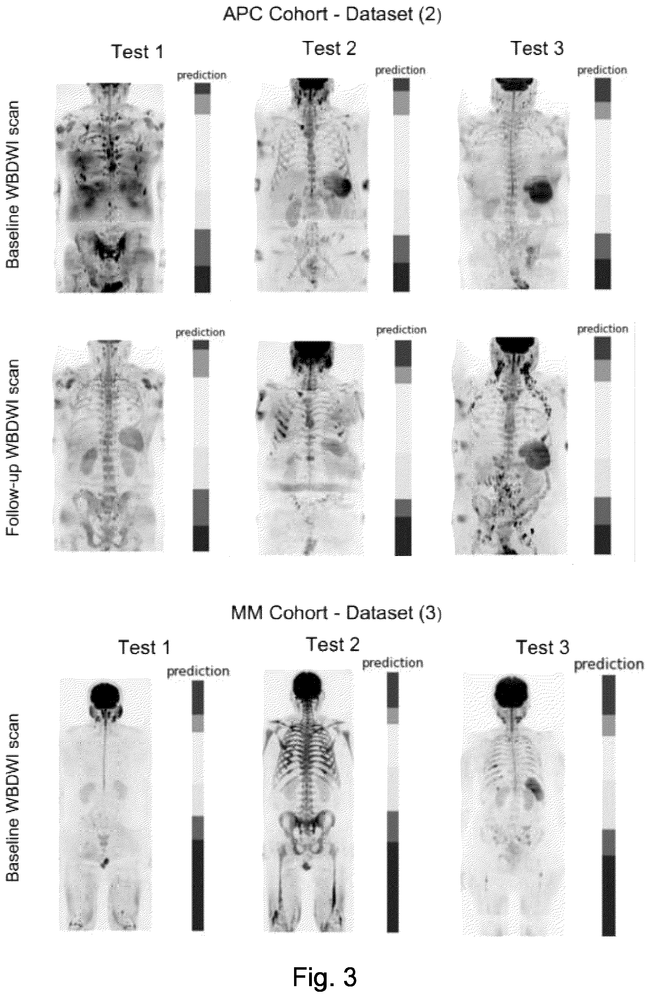

**Figure 3****.** Coronal view of Maximum-Intensity-Projection (MIP) of b = 900 s/mm² image and map of predicted slice's position for six body regions within the skeleton (from bottom to top: legs, pelvis, lumbar/thoracic/cervical spine and head - the vertical extent of each region being indicated by a respective grey scale on the right hand bar) for six test datasets: three patients with confirmed MM from Dataset (2) and three patients with confirmed APC from Dataset (3). Excellent agreement patient-wise is shown between the region classifier predictions and true position of axial images.

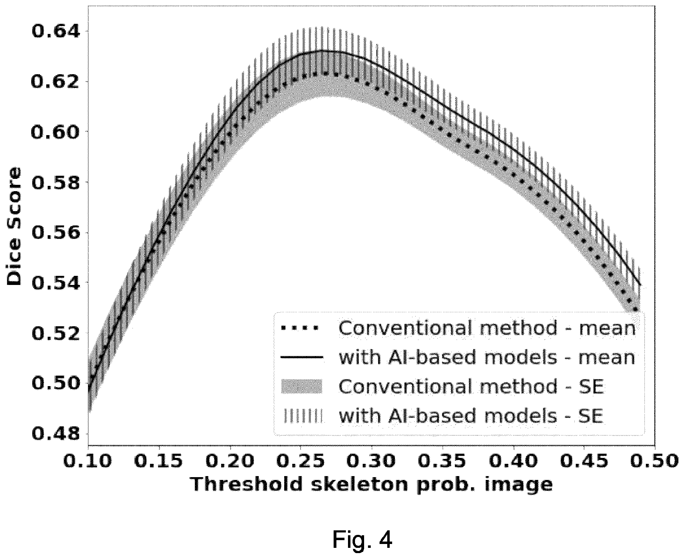

**Figure 4****.** Mean (solid and dotted lines) and standard error (hatched and shaded areas) of dice score against threshold for all test data in the atlas cohort for each of (i) manual segmentation versus Al-assisted atlas-based segmentation, and (ii) manual segmentation versus conventional atlas-based segmentation. The skeleton segmentation results from thresholding the respective skeleton probability image. Both the Al-assisted and the conventional approaches show the best dice score for a threshold of 0.265.

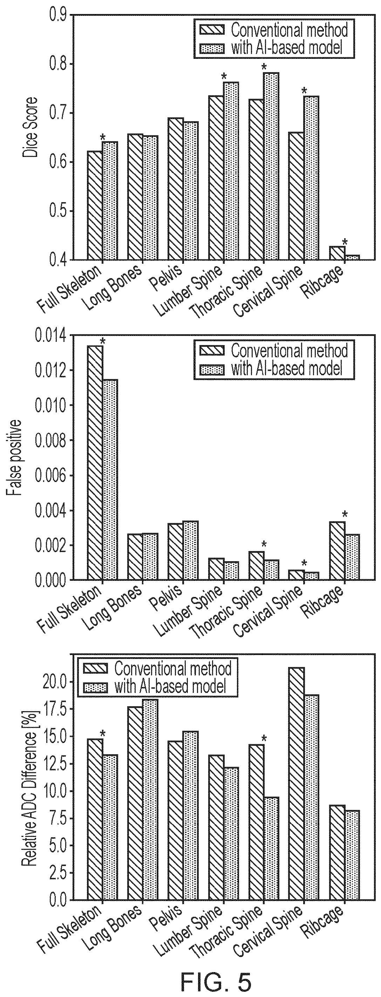

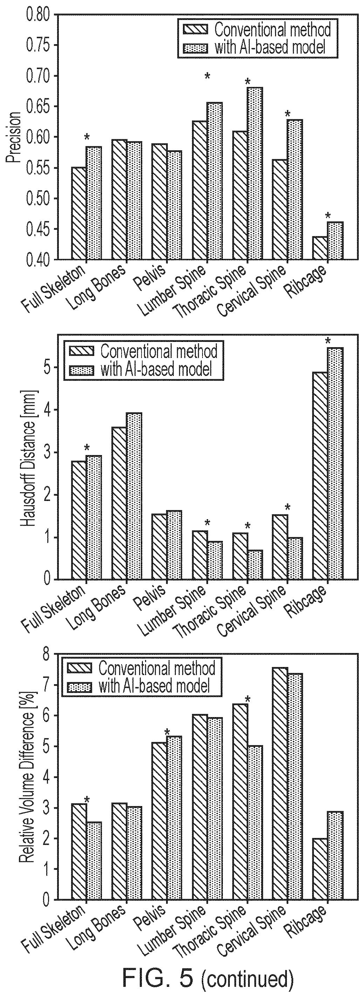

**Figure 5****.** Metrics and parameters used for assessing the performance of the registration method with/without initialisation to delineate the full skeleton, long bones, pelvis, lumbar, thoracic, cervical spine and ribcage for all test data in the atlas cohort. Atlas-based registration with Al-based initialisation showed superior values and statistical difference for all metrics and parameters (except average Hausdorff distance) and outperforms the conventional method in segmenting the spine. Same performance for long bones, pelvis and ribcage. Higher values for relative median ADC and volume difference were observed for the conventional method. Significant differences between the conventional method and registration with Al-based models are indicated withy asterisks (p<0.05, Wilcoxon paired rank-sum test: two-tailed).

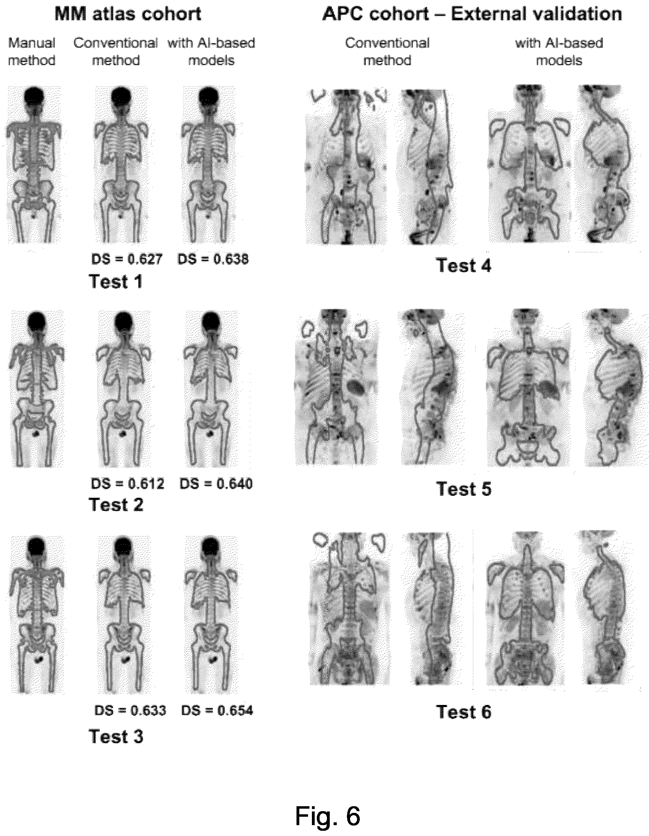

**Figure 6****.** Coronal and sagittal view of Maximum-Intensity-Projection (MIP) of b = 900 s/mm² image and superimposed skeleton segmentation derived from the atlas-based registration with/without Al-based initialisation for three patients with confirmed MM **(Dataset (4),** atlas cohort) and three patients with confirmed APC **(Dataset (3),** external validation). WBDWI scans were acquired from two different sites.
The conventional method shows biased and skewed skeleton segmentations that might include soft tissue, missing bone lesions and bias the ADC measures. Instead, the atlas-based registration with Al-based models shows accurate skeleton segmentations on the same test datasets. The initial deformation derived from the Al-based models transforms the atlas image closer to the target image and overcomes the limitations of the conventional method.

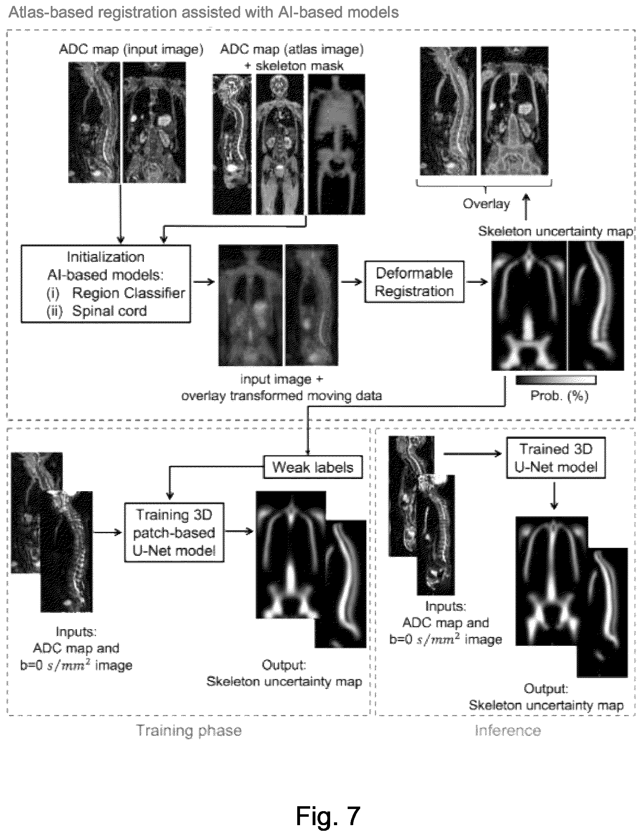

**Figure 7****.** Development and use of a deep learning model that automatically delineates the skeleton from WBDWI. Atlas-based registration assisted with Al-based models was used to generate skeleton uncertainty maps. These images were used as "weak labels" for training a 3D patch-based U-Net model with 2-channel input: the ADC map and b=0 s/mm² image. Inference using the trained U-Net model was performed (without any registration step) in less than 5s.

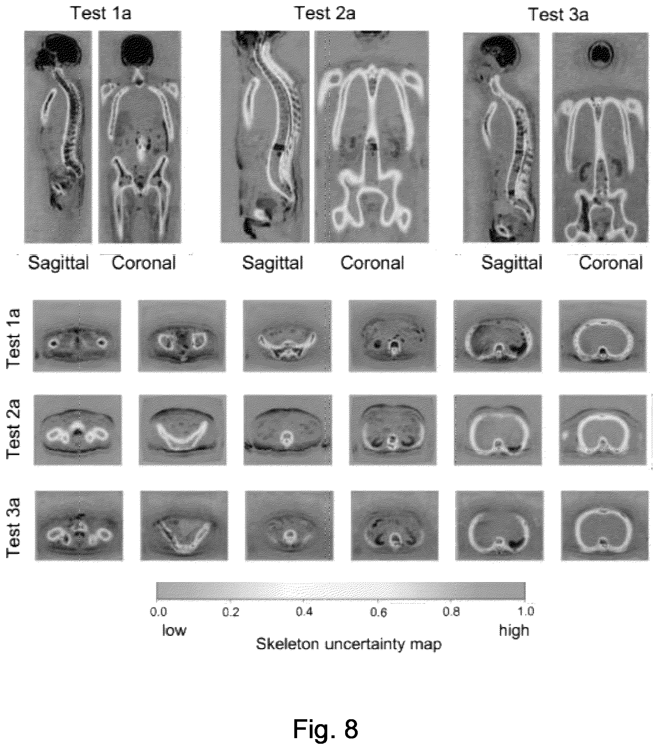

**Figure 8****.** Skeleton uncertainty maps derived from the trained 3D U-Net model superimposed on axial, sagittal and coronal b=900 s/mm² images for three cases in the validation dataset. Smooth maps were derived from the deep learning model showing low and high uncertainty within the patient skeleton and soft tissue background, respectively.

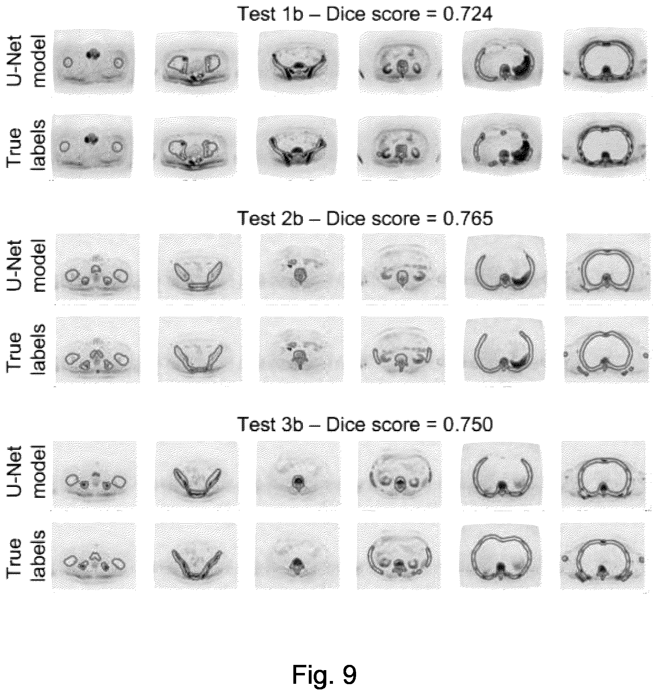

**Figure 9****.** Axial b=900 s/mm² images and superimposed manual (green) and derived skeleton mask (red) for three cases in the atlas cohort, **Dataset (4).** Skeleton masks (red) were obtained by applying a threshold of 0.45 to the derived skeleton uncertainty maps from the trained 3D U-Net model. Good agreement between manual and derived skeleton masks was observed for different skeleton regions. The deep learning model removes with good accuracy the soft tissue background.

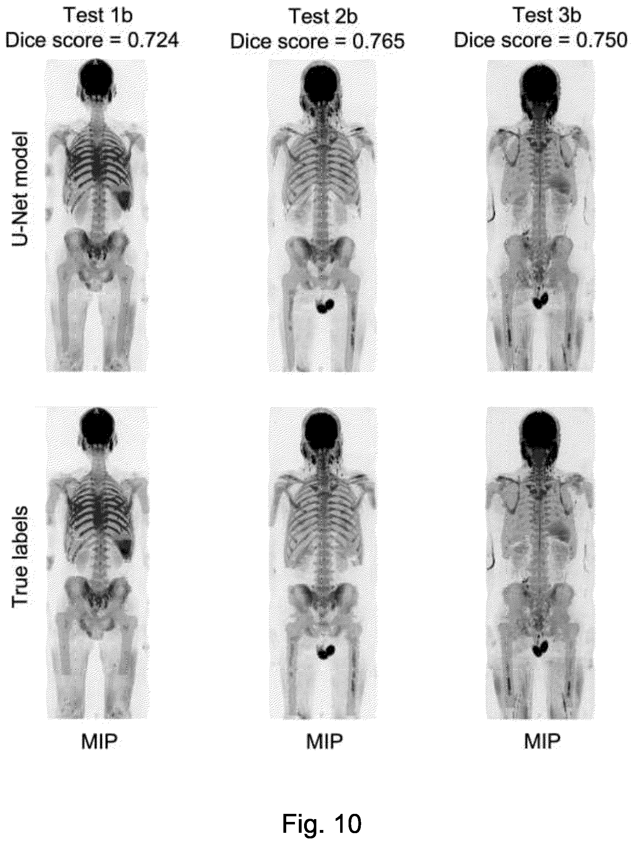

**Figure 10****.** Maximum-Intensity-Projection (MIP) of b=900 s/mm² images and superimposed manual (bottom row) and derived skeleton mask (top row) for three cases in the atlas cohort, **Dataset (4).** Skeleton masks (top row) were obtained by applying a threshold of 0.45 to the derived skeleton uncertainty maps from the trained 3D U-Net model. Good agreement between manual and derived skeleton masks was observed for the legs, pelvis, and spine. Less accurate segmentations were observed for clavicles, shoulders, and upper arms.

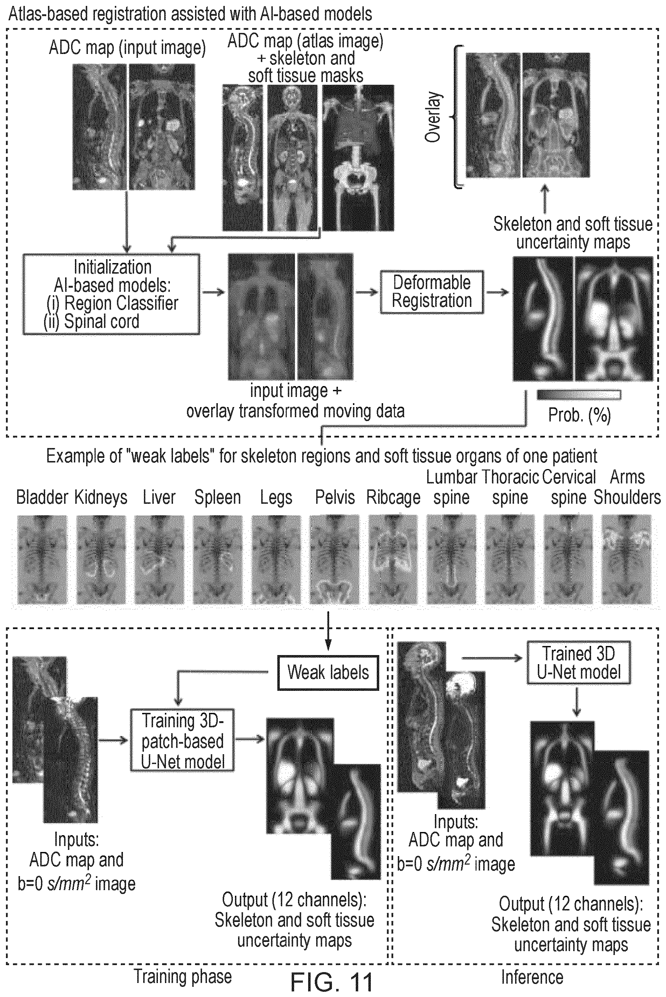

**Figure 11****.** Development and use of a deep learning model that automatically identifies and delineates seven skeleton regions and four soft tissue organs from WBDWI. Atlas-based registration assisted with Al-based models was used to generate the body region uncertainty maps. These images were used as "weak labels" for training a further 3D patch-based U-Net model with 2-channel input, the ADC map and b=0 s/mm² image. Inference was performed using the trained U-Net model without any registration step in less than 20s. The output consisted of 12 channels.

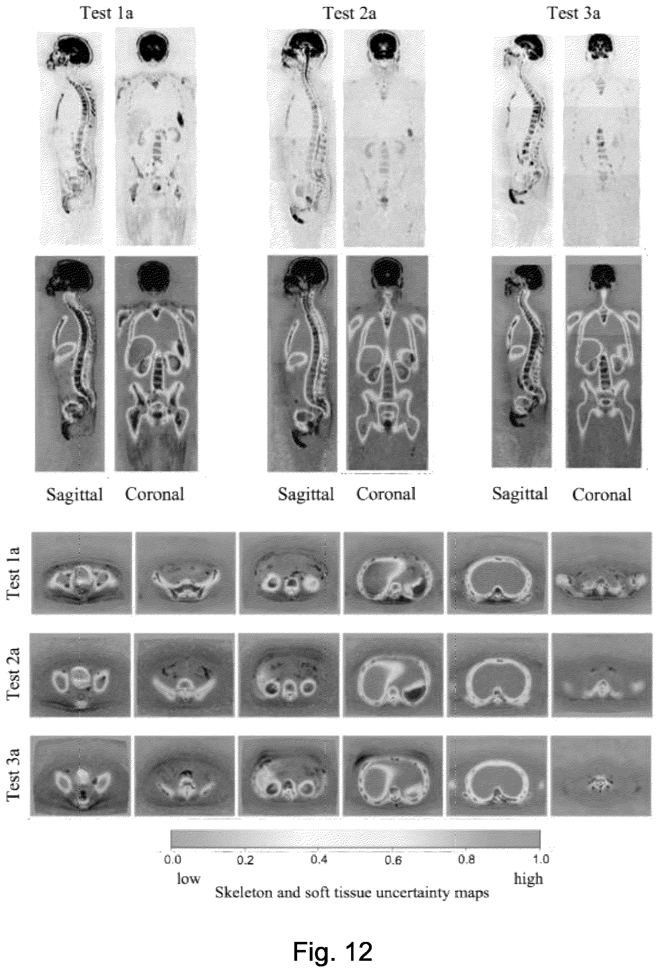

**Figure 12****.** Skeleton and soft tissue uncertainty maps derived from the trained further 3D U-Net model superimposed on axial, sagittal and coronal b=900 s/mm² images for three cases in a validation dataset. The uncertainty maps follow the actual patient anatomy with low uncertainty values within the skeleton and soft tissue organs and high uncertainty values within the image background.

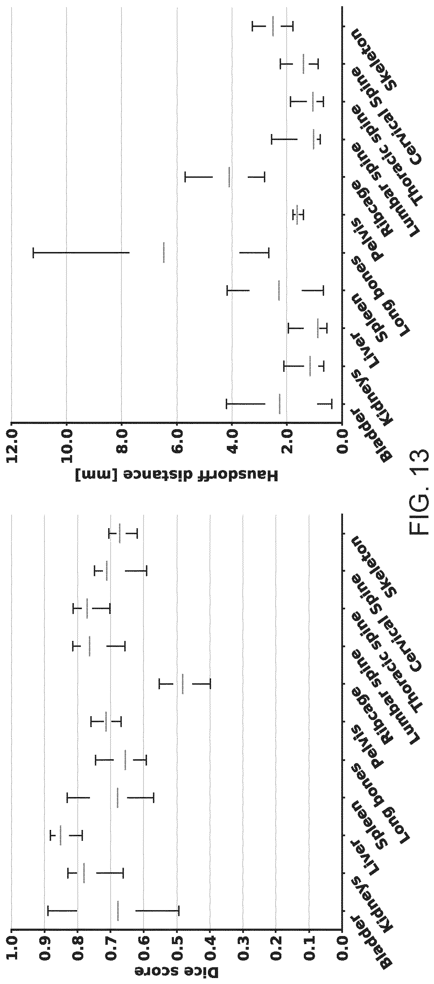

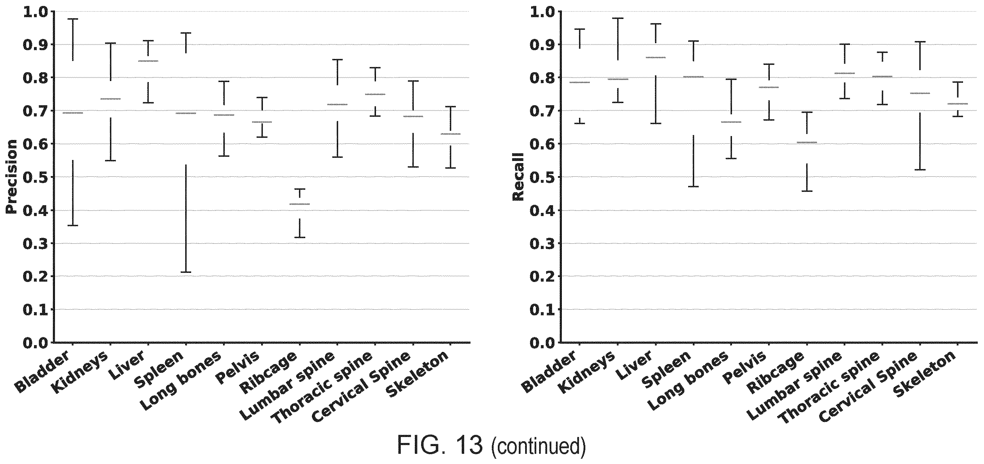

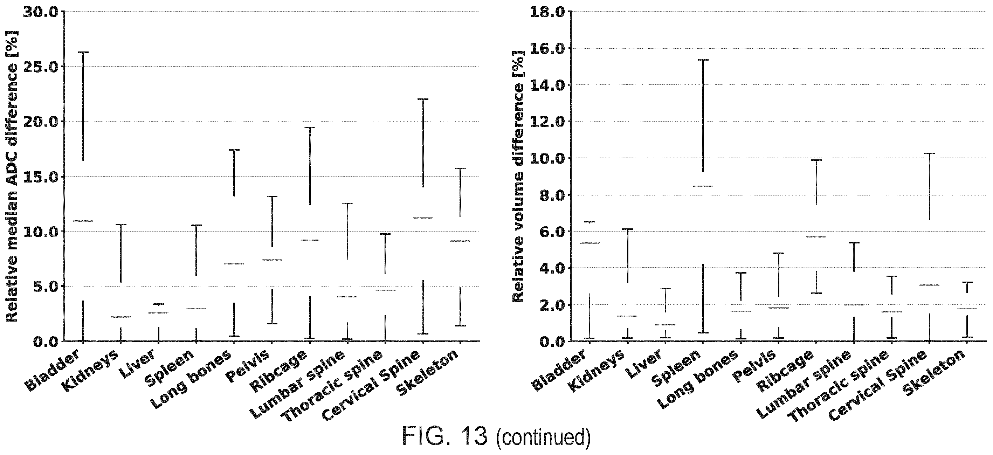

**Figure 13****.** Performance metrics for assessing the segmentation accuracy of the trained further 3D U-Net model compared with manual expert's body region delineations in the atlas cohort, **Dataset (4).** Metrics are reported for the soft tissue organs, the skeleton regions, and the whole skeleton. Mask for long bones consisted of legs and arms/shoulders.

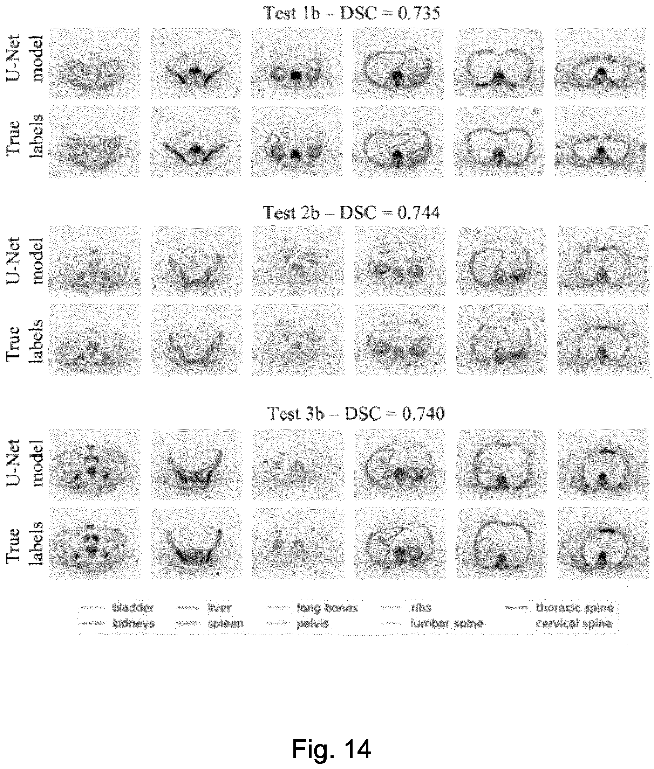

**Figure 14****.** Axial b=900 s/mm² images and superimposed manual and derived skeleton and soft tissue masks for three cases in the atlas cohort, **Dataset (4).** Binary masks for each body region were obtained from thresholding the body region uncertainty maps from the trained further 3D U-Net model. Good agreement between manual and automated methods was observed for different skeleton regions and soft tissue organs. The deep learning model removes the image background, as expected. The DSC was calculated considering all body regions from both methods as a single binary mask.

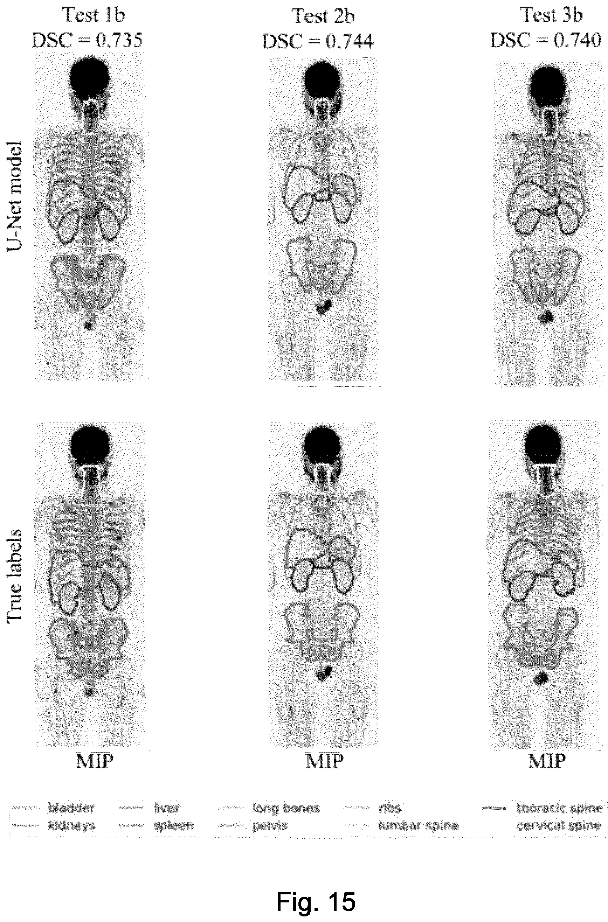

**Figure 15****.** Maximum-Intensity-Projection (MIP) of b=900 s/mm² images and superimposed manual and derived skeleton and soft tissue organ masks for three cases in the atlas cohort, **Dataset (4).** Binary masks for each body region were obtained from thresholding the body region uncertainty maps from the trained further 3D U-Net model. Good agreement between manual and derived body region segmentations was observed for the legs, pelvis, spine, and soft tissue organs. Less accurate segmentations were observed for clavicles, shoulders, and upper arms. The DSC was calculated considering all body regions from both methods as a single binary mask.

### Detailed Description of the Invention

Aspects and embodiments of the present invention will now be discussed with reference to the accompanying figures. Further aspects and embodiments will be apparent to those skilled in the art.

In the following we describe a first study which adopts an innovative approach to initialise the registration step in atlas-based segmentation of WBDWI using transfer deep-learning, making atlas-segmentation more robust to the potential pitfalls of conventional techniques. The approach implements an automated classification tool for identifying the body region (legs, pelvis, lumbar/thoracic/cervical spine, and head) of each slice within the WBDWI dataset. It also implements a preferred automatic spinal cord segmentation tool for initial alignment of the vertebral bodies in WBDWI.

Although discussed in relation to WBDWI, the approach is applicable to DWI generally.

We then describe a second study which uses a training set of skeleton probability images (i.e. skeleton uncertainty maps or 'weak labels') derived from performing the innovative approach of the first study to train and validate a deep learning model for skeleton delineation of WBDWI. Advantageously, this deep learning model can be trained without further reference to ground truth images.

Following that, we describe a third study which extends the approach of the second study to train and validate a further deep learning model for delineation of soft tissue organs, as well as skeleton regions, from WBDWI.

### First Study Materials and Methods

### Patient Population and Imaging Protocol

The training and validation cohort for developing the Al-based models consisted of 40 patients with confirmed Advanced Prostate Cancer (APC), **Dataset (1).** The external validation cohort consisted of 11 patients with confirmed MM and 22 patients with confirmed APC, **Dataset (2)** and **Dataset (3),** respectively. The atlas patient cohort consisted of 15 patients with confirmed diffuse MM (13 male, 2 female), **Dataset (4).** Patients with confirmed APC underwent baseline and after-treatment WBDWI scans. Patients with confirmed MM underwent WBDWI scan prior to treatment initiation. Images were acquired from two different institutions, Royal Marsden NHS Foundation Trust **(Dataset (1), (2)** and (4)) and Musgrove Park Hospital **(Dataset (3)).**

WBDWI scans were acquired using two or three b-values = 50/600/900 s/mm² on a 1.5T scanner (MAGNETOM Aera/Avanto, Siemens Healthcare, Erlangen, Germany), over 4-5 stations from the skull base to mid-thigh, with each station comprising 40 slices. Echo-planar image acquisition was used (GRAPPA=2) employing a double-spin echo diffusion encoding scheme applied over three orthogonal encoding directions with MRI parameters reported in Table 1 for each dataset involved in the study.

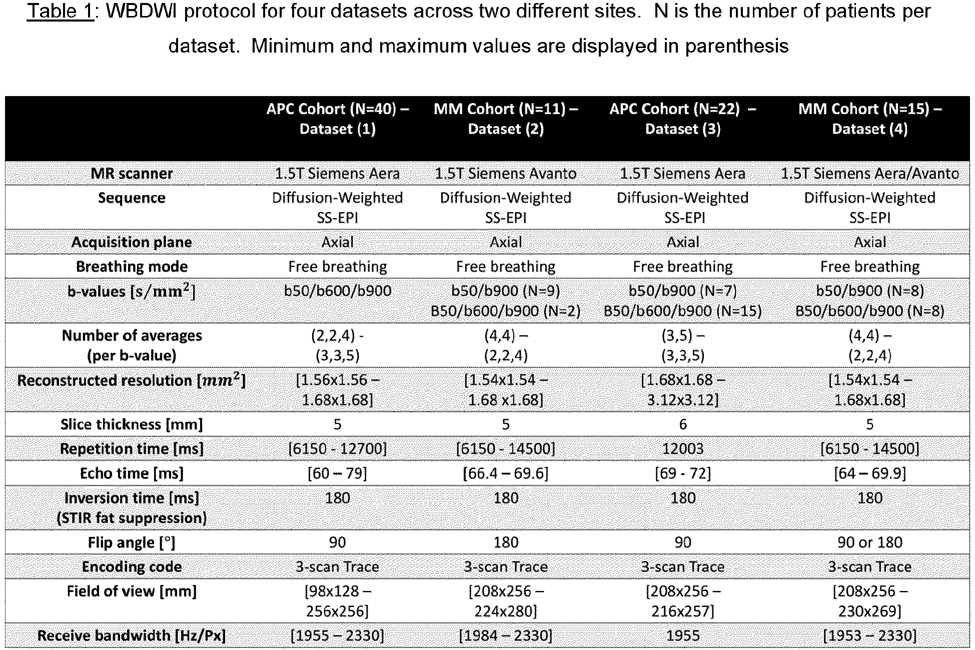

All data were fully anonymised, and the study was performed in accordance with the Declaration of Helsinki (2013). A local ethical committee waived the requirement of patient consent for use of these retrospective datasets.

### Spinal cord delineation

A U-net deep learning model [25] was trained to automatically segment the spinal cord on WBDWI. The network involves a 2-channel input (i) the ADC map (including negative values), and (ii) the b = 0 s/mm² (S0) image, both of which were generated by fitting a monoexponentially decay model to the b-value data [26], [27]. The single-channel output consisted of the probability map of the spinal cord. Ground truth was defined by a medical physicist (>2 years' experience with WBDWI) who manually delineated the spinal cord on WBDWI. All images were interpolated to matrix = 256 x 256 and resolution = 1.6 x 1.6 mm. Input images were normalised using the following transformations: $scaled ADC map = ADC map / 3.5 x {10}^{- 3} {mm}^{2} / s$ (1) $scaled S 0 image = log \left(S0 image\right) / max \left(log \left(S0 image\right)\right)$ (2)

All convolution layers employed 32 filters, 3x3 kernel size, batch normalisation and ReLu activation function except for the final output layer which employed a sigmoid activation function. Adam optimisation (learning rate 10⁻³) was used to minimise a focal Tversky loss function [28] over 150 epochs using a batch size of 8. All algorithms were implemented using Tensorflow 2.3.1 and Keras toolboxes and trained using NVIDIA RTX6000 GPU (Santa Clara, California, US).

Patient data from **Dataset (1)** were split into training, validation and test datasets: the number of patients used for training/validation was 32, whilst 8 patients were held back for testing. All available axial slices from the 32 training/validation patients were processed resulting in a total of training and validation size of 8749 and 2758, respectively (70:30 split). Similarly, a total of 2687 2D images were extracted from the test cohort. A threshold of 0.5 was applied to the spinal cord probability map to derive a binary mask.

To evaluate performance of the U-net model, for each patient in the test cohort, the dice similarity coefficient, precision and recall were calculated both the manual and automatically generated spinal cord segmentations. Qualitative external validation of this algorithm was subsequently performed on 11 patients from **Dataset (2)** and 22 patients from **Dataset (3).**

### Region classifier

A deep learning model was trained for predicting whether a particular axial slice from WBDWI belongs to one of six distinct body regions (legs, pelvis, lumbar/thoracic/cervical spine and head). A transfer learning technique was used, which allows pre-trained models developed for one task being reused as a starting point for a model intended for a different task (so-called "domain adaptation") [29]. The model employed the pre-trained weights of the VGG16 model [30] up to, but not including the final layer, such that it encodes input images (with red, green and blue colour channels) as a 1024-length vector. For our purposes, the channels consisted of the calculated S0 image (red), the estimated ADC map (green), and an array of zeros in the final channel (blue). The resultant 1024-length vector is then fed into a second fully connected neural network with 1024 input nodes and 6 output nodes (the latter using a softmax activation). The weights of the fully connected network were trained using the backpropagation algorithm such that the network could accurately predict body region of an input WBDWI slice. Finally, a sagittal median filter with a 5x5 kernel was used to correct minor slice misclassifications and improve classifier performance for each patient.

The ground truth was determined by a medical physicist with more than 2 years' experience in whole-body MRI. Slices that belong to head data were identified as any images superior to the base of the brain. Slices that belong to the cervical, thoracic and lumbar spine were identified according to the presence of vertebral bodies (C1 to C7, T1 to T12, and L1 to L5 respectively). For the slices inferior to this, any images superior to the base of the pubic rami were classified as pelvis, and any inferior slices were classified as legs. To ensure that all images were compatible with the VGG16 network, they were interpolated to matrix size 128 x 128 and resolution = 2.5 x 2.5 mm (zero padding was used for regions that the original image did not extend to). Furthermore, all images were scaled to ensure values within range [0,1] following equations (1) and (2).

Adam optimisation (learning rate 10⁻³) was used to train the model by minimising the categorical cross-entropy loss function over 100 epochs using a batch size of 256. The networks were implemented using Tensorflow 2.3.1 and Keras toolboxes and trained using NVIDIA RTX6000 GPU (Santa Clara, California, US).

Training and validation were performed using all patients (32:8) from **Dataset (1).** To evaluate region classifier performance, confusion matrix and overall accuracy axial-wise were derived between the ground truth and predictions of slices belong to one of six regions on test datasets. Furthermore, qualitative external validation was performed using all patients from **Datasets (2)** and **(3).**

### Atlas-based segmentation of the skeleton

Registration algorithms with/without initialisation from the above-mentioned Al-based models (i.e. spinal cord delineation and region classifier) were investigated to derive the full skeleton segmentation on WBDWI. Our atlas comprised of 15 patients with diffuse MM **(Dataset (4))** for which the entire skeleton was manually delineated by an expert radiologist with 3 years of experience. Different labels were used for long bones, pelvis, lumbar/thoracic/cervical spine and ribcage so that regional segmentation could be performed. Segmentation of a patient dataset then consisted of spatial registration of each of the atlas ADC maps ("atlas data") to the full-body ADC map of the novel patient ("novel data"). An overview of our atlas-based segmentation pipeline is presented in Figure 1, which consists of four steps:
1. Data preparation
   1.1. The spinal cord on both novel and atlas datasets was segmented using the spinal cord delineation UNET model.
   1.2. Axial slices in both novel and atlas datasets were classified according to body region using our transfer-learned region classifier.
   1.3. A linear model was derived between the central craniocaudal positions of four of the body regions on both atlas and novel datasets (cervical, thoracic, lumbar and pelvis). The positions of head and leg regions were excluded in this calculation as the coverage of both regions may vary between patients and is often not complete.
   1.4. Novel and atlas ADC maps were resampled to an isotropic resolution of 7 mm. This resolution represented a trade-off between computational time and accuracy of subsequent spatial registration.
2. Initial slice-wise registration
   2.1. The linear model derived from step 1.3 was used to initially align the atlas and novel ADC maps along the craniocaudal direction (allowing both translation and stretching along this direction); the atlas data were resampled to match the resolution and field of view of the novel dataset.
   2.2. Then, for each axial slice, the atlas ADC maps were translated in-plane such that the centroid of the spinal cord segmentation from step 1.1 matched that from the novel dataset.
   2.3. This was followed by affine registration in-plane for each axial slice of the ADC maps using a mean-square-error (MSE) cost function.
3. Full-volume deformable registration
   3.1. To mitigate inter-patient anatomical variability, 3D affine followed by diffeomorphic demons registration [31] was performed between each full-body atlas ADC volume and the novel ADC volume (MSE cost used) to provide a final registration transformation of each full-body atlas ADC volume that matches the novel ADC volume.
   3.2. The final MSE cost between each atlas dataset and the novel dataset was stored to quantitatively assess the accuracy of the 3D registration.
4. Skeleton probability image
   4.1. Steps 1-3 were repeated for all 15 atlas datasets, and in each case, the final registration transform derived from ADC maps was applied to the manually annotated skeletal segmentation.
   4.2. A weighted average of the transformed atlas masks (with weights equal to the reciprocal of the MSE values obtained in step 3.2) was used to obtain a probability map defining the location of the skeleton on the novel dataset.

Registration algorithms were implemented using the open-source software Insight Toolkit (ITK, https://itk.org/) version 4. Affine registration was performed using an amoeba optimizer (Simplex delta = 0.005) with linear interpolation, and employing a multi-resolution approach for fully 3D registrations with shrink factors and smoothing sigmas of (4,2,1) and (4,2,0) respectively. For deformable demons registration, Gradient Descent optimization was performed (learning rate = 2) using linear interpolation with multi-resolution non-rigid shrink factors, smoothing sigmas and a convergence window size of (4,2,1), (4,2,0) and 20, respectively. Convergence tolerance and number of iterations for both registration methods were 1e-6 and 200, respectively.

### Evaluation Criteria

Leave-one-out cross validation (LOOCV) was performed within the atlas dataset to evaluate the performance of skeletal segmentations derived from the atlas-based algorithm (performance measured for each of the 15 patients within this cohort, when using the remaining 14 patients as the atlas). Performance metrics were derived by comparing the atlas-based segmentation with the manual contour and included (i) the Dice similarity coefficient (DSC), (ii) precision, (iii) false positive rate, (iv) Hausdorff distance, (v) median ADC value, and (vi) skeletal volume (following logarithmic transform) [32]. To optimise the probability threshold for creating a binary skeleton mask, the DSC from LOOCV was evaluated over a range of thresholds between 0 and 0.5; the maximum DSC provided the optimal threshold for further evaluation.

We compared LOOCV performance when using the previously-mentioned atlas-based segmentation technique including Al initialisation (hereafter "Al-assisted atlas-based segmentation"), with atlas-based segmentation derived when steps 2.1-2.3 were replaced with a conventional initialisation step of matching the centre of mass between each of the atlas ADC volumes and the novel ADC volume (hereafter "conventional atlas-based segmentation"). Significant differences in the performance of Al-based and conventional initialisation methods were assessed using a Wilcoxon signed-rank test (p < 0.05 indicating significance).

Finally, qualitative visual assessment of the full skeleton segmentations derived from the manual conventional atlas-based and Al-based atlas-based segmentations was performed on external WBDWI test datasets (Datasets (2) and (3)).

### First Study Results

### Spinal cord delineation

The U-net model showed high accuracy in predicting the spinal cord on WBDWI. On average the dice score, precision and recall patient-wise on test datasets were 0.88/0.89/0.90, respectively. Furthermore, the model showed reliable segmentations on external WBDWI test datasets. Figure 2 shows the derived spinal cord segmentations on six test data, three from **Dataset (2)** and three from **Dataset (3),** randomly selected. For a qualitative assessment, the segmentations were superimposed on sagittal Maximum-Intensity-projection (MIP) of b = 900 s/mm² images resulting in good agreement with the spinal cord position.

### Region classifier

Table 2 shows the confusion matrix with predictions and true values for all slices in the test datasets. The region classifier misclassified a limited number of slices resulting in an overall accuracy of 0.92.

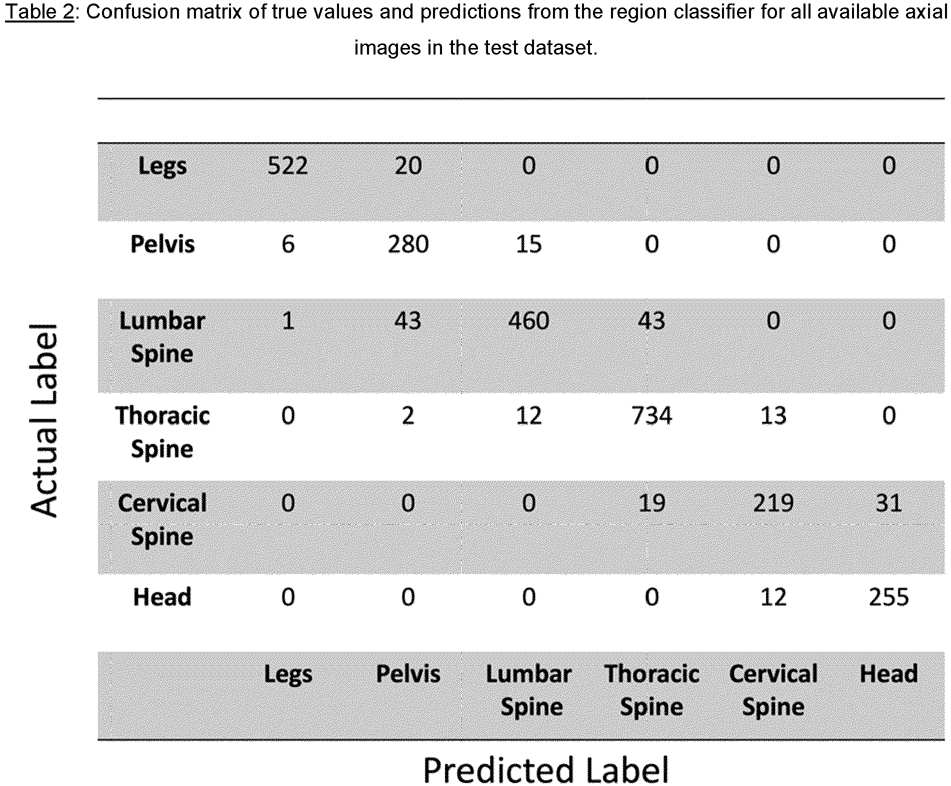

To qualitatively assess the model performance, Figure 3 shows the predictions of slices belong to one of six regions within the skeleton aligned with the coronal MIP of b = 900 s/mm² images for six test datasets, three from **Dataset (2)** and three from **Dataset (3),** randomly selected. The region classifier showed excellent generalization on external test datasets and the results proved how the model can be used to identify with low uncertainty the position (and single images) of legs, pelvis, lumbar/thoracic/cervical spine and head patient-wise on WBDWI.

### Atlas-based segmentation of the skeleton

Figure 4 demonstrates the DSC for the LOOCV experiment of the atlas dataset over a range of probability thresholds. Registration with Al-based models showed a slight improvement in segmenting the full skeleton on WBDWI compared with the manual segmentation (on average dice score was 0.64 and 0.62 (p=0.02), respectively). Moreover, both methods showed the same optimal probability threshold (0.265).

Figure 5 shows the performance criteria derived from the LOOCV analysis for both conventional atlas-based segmentation and Al-assisted atlas-based segmentation. Average Hausdorff distance was lower than 3 mm, relative median ADC difference lower than 13.5%, relative volume difference lower than 3.25%, precision and recall between 0.55 and 0.72 for both methods. However, registration with Al-based models demonstrated significantly improved performance across all metrics other than the average Hausdorff distance. When comparing the segmentation performance for individual skeletal regions, performance was higher in the spine (average dice score for lumbar/thoracic/cervical spine of 0.76/0.78/0.73) and generally good in the long bones and pelvis (average dice score of 0.69 and 0.65, respectively). Moreover, ribcage suffered from markedly poorer performance in terms of DSC, precision and Hausdorff distance, yet demonstrated good performance for evaluating rib volume and median ADC value. In the majority of cases, the Al-assisted atlas-based segmentation method outperformed the conventional approach.

Figure 6 shows the full skeleton segmentations for three patients from the LOOCV analysis and three patients from the external test WBDWI datasets. For qualitative assessment, the segmentations were superimposed on coronal/sagittal MIP of b = 900 s/mm² images. For the external validation datasets, it is observed that the Al-assisted atlas-based segmentation significantly outperformed the conventional atlas-based segmentation and improved generalisation.

A significant difference in computational time was observed among the registration methods to derive the skeleton segmentation across all the test datasets. Using a 2.4 GHz Quad-Core Intel Core i5, the computational time of the conventional atlas-based segmentation was on average 6±0.33 minutes while the computational time for the Al-assisted atlas-based segmentation was on average 4.25±0.31 minutes (including computational time for inference of two Al-based models).

### Second Study Materials and Methods

### Patient Population and Image Acquisition

For training the deep learning model two retrospective WBDWI cohorts were used: 200 patients diagnosed with APC, **Dataset (5),** and 46 patients diagnosed with MM, **Dataset (6).** Every patient from the APC cohort underwent baseline and post-treatment WBDWI scans acquired from three imaging centres (169/22/9 patients per centre). Patients with confirmed MM underwent only the baseline WBDWI scan acquired from two imaging centres (32/14 patients per centre).

Skeleton uncertainty maps (probability images) used for training the supervised deep learning model were derived from the atlas-based registration approach of the first study described above. As already mentioned, the atlas comprised 15 single centre WBDWI scans of patients with diffuse MM, **Dataset (4),** for which the entire skeleton was manually delineated by an expert radiologist with 3 years of experience in whole-body MRI.

WBDWI scans were acquired using two (50/900 s/mm²) or three (50/600/900 s/mm²) b-values on a 1.5T scanner (MAGNETOM Aera/Avanto, Siemens Healthcare, Erlangen, Germany), over 4-5 stations from the skull base to mid-thigh (APC) or skull vertex to knees (MM), with each station comprising 40 slices. Echo-planar image acquisition was performed (GRAPPA=2) using a double-spin echo diffusion encoding scheme applied over three orthogonal encoding directions.

### Deep Learning Model

Patient data from **Datasets (5)** and **(6)** were split 80:20 into a training set and validation set. The data consisted of skeleton uncertainty maps derived from the atlas-based registration approach, and their corresponding ADC maps and estimated b=0 s/mm² (S0) images.

A 3D patch-based U-Net model was trained using the data of the training set. The neural network had a 2-channel input for the ADC map and the estimated b=0 s/mm² (S0) image. Convolution layers of the U-Net model employed 16/32/64/128/256 filters, 3x3 kernel size, batch normalisation, two residual units, dropout rate of 0.3 and PReLu activation function expects for the last layer that employed a sigmoid activation function. Adam optimisation (learning rate 10⁻³) was used to minimise a binary cross-entropy loss function over 150 epochs using a batch size of 4 and patch size of 128x128x64.

The trained U-Net model was then able to automatically generate a skeleton uncertainty map from an ADC map and a corresponding S0 image. The relation of the deep learning model training to the atlas-based segmentation pipeline of the first study described above, and subsequent inference using the trained model are presented in Figure 7.

### Second Study Results

Figure 8 shows the skeleton uncertainty maps derived from the U-Net model for three cases in the validation dataset. Voxels classified with low and high uncertainty aligned with the patient skeleton and soft tissue background, respectively. Inference times of less than 10s were achieved on our CPU (2.4 GHz Quad-Core) compared to on average 4 minutes for the atlas-based registration.

To quantitatively assess the accuracy of the U-Net model, the skeleton uncertainty maps for all the images in the atlas cohort, **Dataset (4),** were derived. A threshold of 0.45 was applied to derive a skeleton mask. The mean dice score, precision and recall between the manual and derived skeleton mask were 0.74, 0.78, and 0.7 (with true labels dilated using a 3x3 structuring element), respectively.

Skeleton masks were transferred into the corresponding ADC map to derive the ADC statistics and volume within the delineated regions. The relative error of median ADC and volume between manual and automated methods was 3.27% and 10.9%, respectively.

Figures 9 and 10 show manual and derived skeleton masks superimposed on axial and Maximum-Intensity-Projection (MIP) of b=900 s/mm² images for three cases in the atlas cohort.

### Third Study Materials and Methods

### Patient Population and Image Acquisition

For training the further deep learning model the same two retrospective WBDWI cohorts were used as for the Second Study, but with more patients set aside for testing. Thus for training, the model used 189 patients diagnosed with APC, **Dataset (5),** and 35 patients diagnosed with MM, **Dataset (6).** Every patient from the APC cohort underwent baseline and post-treatment scans acquired at one of three imaging centres (158/22/9 patients per centre). Patients with confirmed MM underwent a baseline scan at one of two imaging centres (22/13 patients per centre). Patient data from **Dataset (5)** and **(6)** were split 80:20 into training and validation sets.

WBDWI scans were acquired using two (50/900 s/mm²) or three (50/600/900 s/mm²) b-values on a 1.5T scanner (MAGNETOM Aera/Avanto, Siemens Healthcare, Erlangen, Germany), over 4-5 stations from the skull base to mid-thigh (APC) or skull vertex to knees (MM), with each station comprising 40 slices. Echo-planar image acquisition was used (GRAPPA=2) with a double-spin echo diffusion encoding scheme applied over three orthogonal encoding directions.

An ADC map and an estimated intercept (S0) image at b = 0 s/mm² were derived for each patient from **Dataset (5)** and **(6)** by fitting a monoexponentially decaying model to the diffusion data.

Skeleton region and soft tissue organ uncertainty maps (i.e. probability images or 'weak labels') were derived from a weighted atlas-based registration algorithm similar to that of the first study described above. The atlas comprised the same 15 single centre WBDWI scans of patients with diffuse MM, **Dataset (4)** discussed previously in respect of the First Study. However, now the entire skeleton and soft tissue organs were manually delineated by an expert radiologist with over 3 years of experience in whole-body MRI. Different labels were assigned to masks corresponding to eleven body regions, namely: seven skeleton regions (legs, pelvis, lumbar spine, thoracic spine, cervical spine, ribcage and arms/shoulders) and four soft tissue organs (bladder, spleen, liver and kidneys).

### Atlas-Based Registration

A respective non-linear deformable registration was performed between each of the 15 atlas whole-body ADC maps ("atlas data") and the whole-body ADC map ("novel data") for each patient in the training set. Preliminary to the deformable registration, two previously described Al-based models (i.e. region classifier and spinal cord delineation discussed previously in respect of the First Study) were employed to optimize the initial alignment between each atlas data map and each novel data map by (i) automatically classifying image slices into one of five contiguous regions (legs, pelvis and lumbar/thoracic/cervical spine), and (ii) automatically delineating the spinal cord. After this initial alignment, full-volume deformable registration was performed (as discussed previously in respect of the First Study) to derive a transformation of each atlas data map that matches the novel data map. The derived transformations were then applied to all eleven pre-labelled body region masks of each patient. The 15 transformed masks from each region of each patient were combined using a weighted average, fusion label technique to derive the uncertainty map at each skeleton region and soft tissue organ: $uncertainty {map}^{c} = \frac{{\sum }_{i = 1}^{n} {w}_{i} ⋅ T \left({l}_{c}^{i}\right)}{{\sum }_{i = 1}^{n} {w}_{i}}$ (1) where T is the transform derived from the spatial registration between the novel data and atlas data applied to the respective atlas mask *I*; c being the number of body regions to delineate (eleven in this example); n being the number of atlas maps (15 in this example); and w being the mean-squared errors (or weights) derived from affine registrations only.

The computational time for the atlas-based registration was, on average, 5 minutes on CPU (2.4 GHz Quad-Core).

The top part of Figure 11 illustrates schematically how an uncertainty map at the eleven body regions was obtained for each patient in the training set.

### Deep Learning Model

A further 3D patch-based U-Net model was trained to predict skeleton and soft tissue organ uncertainty maps using the training set from the preceding phase. The network had a 2-channel input for: (i) the ADC map of each patient (no thresholding being applied to remove negative ADC calculations) and (ii) the corresponding estimated intercept (S0) image. The U-Net architecture consisted of 5 convolution layers with filters down/up sampled by a factor of 2 at each layer starting from 16. All convolution layers employed 3x3 kernel size, batch normalisation, a dropout rate of 0.2 and the ReLu activation function except for the final, multi-channel, output layer which employed a softmax activation function. Stochastic gradient descent optimisation (learning rate = 0.1, momentum = 0.9 and weight decay = 4 x10⁻⁵) was used to minimise a cross-entropy loss function investigated over 150 epochs using a batch size of 4 and patch size of 128x128x64.

For each patient of the training set, the uncertainty map derived from the atlas-based registration was arranged so that the parts of the map corresponding to the eleven body regions (legs, pelvis, lumbar spine, thoracic spine, cervical spine, ribcage, arms/shoulders, bladder, kidneys, spleen and liver) were stacked to compose an array of "weak labels". In this way, the part of the overall uncertainty map corresponding to each body region provided one channel of the multi-channel output of the further deep learning model. A twelfth channel of the output was reserved for image background, and was derived by subtracting 1 from a voxel-wise sum of the eleven weak labels.

All images were interpolated to matrix = 256 x 256 and resolution = 1.6 x 1.6 mm. Input images were normalised using the following transformations: $scaled ADC map = ADC map / 3.5 x {10}^{- 3} {mm}^{2} / s$ (2) $scaled S 0 image = log \left(S0 image\right) / max \left(log \left(S0 image\right)\right)$ (3)

The algorithms were implemented in Python (v.3.7) using PyTorch v.1.12.1 and MONAI v.0.9.1 toolboxes, running upon a Windows platform (v.10.0.19) accelerated by an NVIDIA RTX6000 GPU (Santa Clara, California, US).

The bottom left part of Figure 11 illustrates schematically the training phase. Once trained the ADC maps and (S0) images of the validation set were input into the further deep learning model to generate skeleton and soft tissue probability images, as illustrated schematically at the bottom right part of Figure 11.

### Third Study Results

Figure 12 shows the uncertainty maps from the multi-channel output of the U-Net model for three cases in the validation dataset. From visual assessment of maximum-intensity-projection (MIP) of high b-value images (b = 900 s/mm²) and superimposed maps, voxels classified with low uncertainty aligned with actual patient skeleton and soft tissue organs, without showing signs of overfitting. Contrarily, voxels that show high uncertainty values belong to the image background, as expected.

The inference time of the trained further deep learning model was below 20 seconds (on CPU 2.4 GHz Quad-Core) per WBDWI scan with an improvement in speed over the atlas-based registration of 93.3%.

To quantitatively assess the performance of the U-Net model from weakly supervised training, the skeleton and soft tissue uncertainty maps for all the images in the atlas cohort, **Dataset (4),** were derived. Overlap and distance-based metrics were obtained by comparing automated segmentations with the manual contours and included (i) the DSC, (ii) precision, (iii) recall, and (iv) average Hausdorff distance. The optimal uncertainty threshold for creating the binary mask for each delineated body region was obtained from the maximum value of the DSC evaluated over a range of thresholds between 0.1 and 0.9.Figure 13 shows a DSC for skeleton segmentation above 0.7 for all regions except the ribcage. Excellent agreement between manual and automated segmentations of kidneys and liver was observed (DSC > 0.75). On average, DSC for the bladder and spleen was less than 0.7, with more variability across test datasets. The number of voxel-wise false negative predictions was lower than false positives, as shown by a higher recall value than precision on test datasets. The average recall value for skeleton and soft tissue organ segmentations was 0.72 and 0.8; precision was 0.62 and 0.73, respectively. Lastly, the average Hausdorff distance was below 3mm for all delineated regions except long bones (legs plus arms/shoulders) and ribcage.

Furthermore, binary masks were transferred into the ADC maps to derive the ADC statistics and volume within the delineated regions (Figure 13). The average relative median ADC and volume difference between manual and automated methods for skeleton segmentation was below 10% and 5%, respectively. These are consistent with an observer repeatability study where inter- and intra-reader repeatability of the same biomarkers was assessed from manual experts' delineations of bone lesions in 10 patients with APC [17]. The relative median ADC difference for soft tissue organs was below 5% except for the bladder, which showed a difference of over 10%. The relative volume difference for the spleen was above 8%, but for the bladder/kidneys/liver was lower than 6%.

Figures 14 and 15 show manual and derived skeleton region and soft tissue segmentations superimposed on axial and MIP of high b-value images for three cases in the atlas cohort. Good agreement between manual and automated segmentations was observed across all skeleton regions and soft tissue organs.

### First to Third Studies - Discussion and Conclusions

Atlas-based registration to derive the full skeleton or other parts of the body on WBDWI is a challenging task. Initial misalignment between the target and atlas image due to complex skeleton structure, inter-patient anatomy variability, and different patient poses during scanning might cause registration failures. Furthermore, WBDWI shows low SNR and high distortion that might amplify the initial misalignment and number of skeleton registration failures. In this study, we have developed an atlas-based registration method that employs two Al-based models to improve the initial alignment between target and atlas images.

Quantitative analysis was performed to assess accuracy in delineating the full skeleton using the Al-assisted atlas-based segmentation compared to manual segmentation and conventional atlas-based segmentation. Across all skeletal regions investigated, our results demonstrated overall better performance by the Al-assisted atlas-based segmentation than the conventional atlas-based segmentation (for the full skeleton test data of the First Study the average dice score, precision, and recall were 0.64, 0.55, and 0.72, respectively), and derivation of biomarker measurements (median ADC and tumour volume) were reasonably accurate for most body regions (relative difference lower than 13.5% and 3.5%, respectively). Cervical spine suffered most extensively in terms of ADC median measurement, which may be attributed to poor geometric fidelity in this region, and an apparent heterogeneity in the pose of the neck between different patients. Moreover, the derived skeleton segmentations showed high precision in removing the soft tissue and background on WBDWI, even when the agreement with the manual method drops. As a result, the Al-assisted registration method might facilitate the automatic bone lesions detection.

The spinal cord segmentations derived on both target and atlas images by the Al-models improve the initialisation by allowing the translation of each vertebra of the atlas image to overlap the pair vertebrae on the target image. More generally, the Al-based initialisation allows a good starting point to be obtained for the subsequent full-volume deformable registration. For example, it is more likely to be unbiased by structures other than bone when the aim of a study is to skeletally segment the patient image by obtaining a skeleton probability image.

Furthermore, qualitative analysis was performed to assess the performance of both the Al-assisted and the conventional atlas-based segmentation on external test datasets. The conventional approach showed biased and skewed skeleton segmentations. There was a significant misalignment between the target and atlas image that cannot be solved by a 3D translation transform initiated from COM of target and atlas image. In contrast, the Al-assisted approach showed reproducible skeleton segmentations even on WBDWI that might show lower SNR and a higher degree of distortion. The region classifier allows aligning of body regions from the atlas image with the corresponding body regions from the target image along the vertical axis prior to deformable registration. Therefore, the region classifier overcomes the weakness of the convention approach and plays a significant role in improving generalisation of the skeleton spatial registration on WBDWI.

The Al-assisted atlas-based segmentation showed similar performance to atlas-based registration algorithms developed to derive skeleton segmentation on high-resolution images (CT, T1w and T2w) published in the literature [21]-[23]. However, the Al-assisted approach employs spatial registration between ADC maps with a computational time on average lower than 5 minutes. The region classifier, particularly when supplemented by the spinal cord model for axial slice in-plane translation, provides a good initialisation for the registration algorithms, overcoming many of the weaknesses of conventional methods for registering ADC maps. In particular, the initial deformation can accelerate convergence of the registration process, with significant improvements in terms of efficiency, accuracy and robustness. The number of cohorts, patients and WBDWI scans employed for testing the registration method in combination with Al-based models was larger and more diverse than any other study in the literature.

The derived skeleton probability image can be combined with the computed b = 900 s/mm² images that shows an excellent contrast to detect normal and diseased bone (appears hyper-intense compared with normal background). A signal-based threshold or supervised machine learning model (that involves a 2-channel input (i) the derived skeleton probability image, and (ii) the computed b = 900 s/mm² image) can thus be applied to delineate ROIs that show same properties that bone lesions, high signal intensity and are located within the derived skeleton segmentation. The aggregate volume of all segmented high signal intensity ROIs on the high b-value DWI is the TDV, which might reflect the disease burden across the whole body. The same ROIs can be transferred onto the derived ADC map to derive a global measurement (such as average ADC), which is considered to be inversely correlated with cellularity in different tumour types. Changes in ADC and TDV after treatment may be used to assess treatment response of metastatic bone disease, thus providing two quantitative metrics from a single radiological investigation [36].

As demonstrated in the Second Study, the skeleton probability images derived from the Al-assisted atlas-based segmentation can be employed for training a 'weak' supervised deep learning model to improve even further the accuracy of full skeleton segmentation on WBDWI. 'Weak' supervision is a branch of machine learning that allows development of a strong predicting model using noise, limited or imprecise sources of supervision [34], [35]. This method reduces or avoids the burden of obtaining large hand-labelled datasets. For example, manual contouring of skeleton masks across large datasets is generally impractical due to lack of time or resources for this task.

Thus, the Al-assisted atlas-based segmentation showed reliable and reproducible skeleton segmentation across diverse DWI cohorts for patients with confirmed MM or APC. The registration method can be employed for developing an automated tool for delineating bone disease on WBDWI. Moreover, skeleton probability images obtained using the atlas-based registration approach can then be used to train a deep learning model which is able to automatically delineate the skeleton from WBDWI without further reference to the atlas. This model also showed similar performance to algorithms developed to derive skeleton segmentation from high-resolution images published in the literature, but with computational times that were reduced by about a factor of 60 relative to the atlas-based registration approach.

Indeed, as demonstrated in the Third Study, the approach used to develop the automated tool can be extended, and we have trained and validated a further deep learning model to automatically identify and delineate skeleton regions and soft tissue organs from WBDWI without further reference to the atlas. Body region masks for training datasets were derived from the atlas-based registration method. The computational time for executing the registration algorithms was above 5 minutes. In contrast, the trained U-Net model performed the same segmentations 15x faster and with superior segmentation accuracy. The model showed performance levels that were similar to published examples of atlas-based registration and deep learning algorithms that perform skeleton and soft tissue organ segmentations from high-resolution and high SNR images (CT, T1w and T2w) [21]-[23]. Thus the model can facilitate the delineation of visceral metastases in patients with APC and extramedullary lesions in patients with MM. Suspected soft tissue lesions that appear with high signal intensity and low ADC values outside a predefined mask (which zeros all the axial images above the cervical spine, below the bladder, voxels that belong to derive skeleton and soft tissue organs segmentations, except the liver) can be automatically delineated and consequently imaging biomarkers can be extracted within the same region of interest.

More generally, the trained 3D U-Net model can generate the uncertainty maps for body regions independently from the number of axial images that compose the ADC map and S0 image. As a result, the model can be deployed for delineating skeleton regions and soft tissue organs from diffusion data acquired with a different scanning protocol than whole-body MRI. For instance, a suitable MRI scanning protocol can extend from the thorax to the patient's pelvic area.

Moreover, the atlas-based segmentation phase might facilitate retraining of the model (or transfer learning) to include WBDWI scans acquired from different scanner manufacturers or facilitate Al-based reconstructed methods [37] for boosting performance without allocating resourcing for manually delineating the skeleton regions and soft tissue organs.

Finally, although the First to Third Studies discussed above conveniently made use of atlas registration based on different skeletal regions of the body, the registration algorithms do not necessarily require a skeleton to define different regions. That is, the algorithms can also be employed to register ground truth images to a patient image even when the regions are not defined by a skeleton, but are merely contiguous anatomical regions of the body; the registration algorithm quantifies the degree of similarity between intensity patterns in the target and atlas image. The deep learning model can predict the spatial positions of these continuous anatomical regions of the body, improve the spatial alignment between atlas and target image and consequently decrease the level of uncertainty in registering the atlas and target images. It can also perform segmenting predictions based on discovered patterns and features from the images that are unique to specific weak labels even when skeletons are not present.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### References

A number of publications are cited above in order to more fully describe and disclose the invention and the state of the art to which the invention pertains. Full citations for these references are provided below.
[1] A. R. Padhani et al., "METastasis Reporting and Data System for Prostate Cancer: Practical Guidelines for Acquisition, Interpretation, and Reporting of Whole-body Magnetic Resonance Imaging-based Evaluations of Multiorgan Involvement in Advanced Prostate Cancer," Eur. Urol., vol. 71, no. 1, pp. 81-92, 2017.
[2] C. Messiou et al., "Guidelines for Acquisition, Interpretation, and Reporting of Whole-Body MRI in Myeloma: Myeloma Response Assessment and Diagnosis System (MY-RADS)," Radiology, vol. 291.1, no. 7, 2019.
[3] R. Perez-lopez and D. Bono, "Imaging Diagnosis and Follow-up of Advanced Prostate Cancer: Clinical Perspectives and State of the Art," Radiology, vol. 292, no. 2, pp. 273-286, 2019.
[4] C. Messiou et al., "Prospective Evaluation of Whole-Body MRI versus FDG PET/CT for Lesion Detection in Participants with Myeloma," Radiol. Imaging Cancer, vol. 3, no. 5, 2021.
[5] R. Donners et al., "Whole-body diffusion-weighted MRI in lymphoma - comparison of global apparent diffusion coefficient histogram parameters for differentiation of diseased nodes of lymphoma patients from normal lymph nodes of healthy individuals," Quant. Imaging Med. Surg., vol. 11, no. 8, pp. 3549-3561, 2021.
[6] M. Morone et al., "Whole-body MRI: Current applications in oncology," Am. J. Roentgenol., vol. 209, no. 6, pp. 336-349, 2017.
[7] F. Z. Id et al., "The added value of whole-body magnetic resonance imaging in the management of patients with advanced breast cancer," PLoS One, vol. 13, no. 10, pp. 1-16, 2018.
[8] H. I. Scher et al., "Trial design and objectives for castration-resistant prostate cancer: Updated recommendations from the prostate cancer clinical trials working group 3," J. Clin. Oncol., vol. 34, no. 12, pp. 1402-1418, 2016.
[9] G. Shen, H. Deng, S. Hu, and Z. Jia, "Comparison of choline-PET/CT, MRI, SPECT, and bone scintigraphy in the diagnosis of bone metastases in patients with prostate cancer: a meta-analysis," Skeletal Radiol., vol. 43, no. 11, pp. 1503-1513, 2014.
[10] D. M. Koh and D. J. Collins, "Diffusion-weighted MRI in the body: Applications and challenges in oncology," Am. J. Roentgenol., vol. 188, no. 6, pp. 1622-1635, 2007.
[11] R. Perez-Lopez et al., "Diffusion-weighted imaging as a treatment response biomarker for evaluating bone metastases in prostate cancer: A pilot study," Radiology, vol. 283, no. 1, pp. 168-177, 2017.
[12] R. Perez-Lopez et al., "Volume of bone metastasis assessed with whole-Body Diffusion-weighted imaging is associated with overall survival in metastatic castration resistant prostate cancer," Radiology, vol. 280, no. 1, pp. 151-160, 2016.
[13] A. Miles et al., "Predictors of patient preference for either whole body magnetic resonance imaging (WB-MRI) or CT/ PET-CT for staging colorectal or lung cancer," J. Med. Imaging Radiat. Oncol., vol. 64, no. 4, pp. 537-545, 2020.
[14] A. R. Padhani, D. M. Koh, and D. J. Collins, "Whole-body diffusion-weighted MR imaging in cancer: Current status and research directions," Radiology, vol. 261, no. 3, pp. 700-718, 2011.
[15] A. Colombo et al., "Semi-automated segmentation of bone metastases from whole-body mri: Reproducibility of apparent diffusion coefficient measurements," Diagnostics, vol. 11, no. 3, 2021.
[16] M. D. Blackledge et al., "Assessment of treatment response by total tumor volume and global apparent diffusion coefficient using diffusion-weighted MRI in patients with metastatic bone disease: A feasibility study," PLoS One, vol. 9, no. 4, pp. 1-8, 2014.
[17] M. D. Blackledge et al., "Inter- and Intra-Observer Repeatability of Quantitative Whole-Body, Diffusion-Weighted Imaging (WBDWI) in Metastatic Bone Disease," PLoS One, vol. 11, no. 4, pp. 1-12, 2016.
[18] M. Cabezas, A. Oliver, X. Lladó, J. Freixenet, and M. Bach Cuadra, "A review of atlas-based segmentation for magnetic resonance brain images," Comput. Methods Programs Biomed., vol. 104, no. 3, pp. 158-177, 2011.
[19] J. E. I. And and M. R. S. Juan, "Multi-Atlas Segmentation of Biomedical Images: A Survey," Med Image Anal., vol. 24, no. 1, pp. 205-219, 2015.
[20] S. D. Almeida et al., "Quantification of tumor burden in multiple myeloma by atlas-based semi-automatic segmentation of WB-DWI," Cancer Imaging, vol. 20, no. 1, pp. 1-10, 2020.
[21] H. Arabi and H. Zaidi, "Comparison of atlas-based techniques for whole-body bone segmentation," Med. Image Anal., vol. 36, pp. 98-112, 2017.
[22] I. Lavdas et al., "Fully automatic, multiorgan segmentation in normal whole body magnetic resonance imaging (MRI), using classification forests (CFS), convolutional neural networks (CNNs), and a multi-atlas (MA) approach," Med. Phys., vol. 44, no. 10, pp. 5210-5220, 2017.
[23] J. Ceranka et al., "Multi-atlas segmentation of the skeleton from whole-body MRI-Impact of iterative background masking," Magn. Reson. Med., vol. 83, no. 5, pp. 1851-1862, 2019.
[24] J. M. Winfield, M. D. Blackledge, N. Tunariu, D. M. Koh, and C. Messiou, "Whole-body MRI: a practical guide for imaging patients with malignant bone disease," Clin. Radiol., vol. 76, no. 10, pp. 715-727, 2021.
[25] O. Ronneberger, P. Fischer, and and T. Brox, "U-Net: Convolutional Networks for Biomedical Image Segmentation," in Navab N., Hornegger J., Wells W., Frangi A. Medical Image Computing and Computer-Assisted Intervention - MICCAI 2015. MICCAI 2015. Lecture Notes in Computer Science, 2015, vol. 9351, pp. 234-241.
[26] M. D. Blackledge et al., "Computed Diffusion-weighted MR Imaging May Improve Tumor Detection," Radiology, vol. 261, no. 2, pp. 573-81, 2011.
[27] M. D. Blackledge et al., "Noise-Corrected , Exponentially Weighted , Diffusion-Weighted MRI ( niceDWI ) Improves Image Signal Uniformity in Whole-Body Imaging of Metastatic Prostate Cancer," Front. Oncol., vol. 10, no. May, pp. 1-12, 2020.
[28] N. Abraham and N. M. Khan, "A novel focal tversky loss function with improved attention u-net for lesion segmentation," 2018.
[29] S. U. Khan, N. Islam, Z. Jan, I. Ud Din, and J. J. P. C. Rodrigues, "A novel deep learning based framework for the detection and classification of breast cancer using transfer learning," Pattern Recognit. Lett., vol. 125, pp. 1-6, 2019.
[30] K. Simonyan and A. Zisserman, "Very deep convolutional networks for large-scale image recognition," in 3rd International Conference on Learning Representations, ICLR 2015 - Conference Track Proceedings, 2015, pp. 1-14.
[31] J. P. Thirion, "Image matching as a diffusion process: An analogy with Maxwell's demons," Med. Image Anal., vol. 2, no. 3, pp. 243-260, 1998.
[32] A. A. Taha and A. Hanbury, "Metrics for evaluating 3D medical image segmentation: Analysis, selection, and tool," BMC Med. Imaging, vol. 15, no. 29, 2015.
[33] R. E. Coleman, Roodman, Smith, Body, Suva, and Vessella, "Clinical features of metastatic bone disease and risk of skeletal morbidity," Clin. Cancer Res., vol. 12, no. 20 PART 2, pp. 6243-6250, 2006.
[34] H. Zheng et al., "Semi-supervised Segmentation of Liver Using Adversarial Learning with Deep Atlas Prior," in Medical Image Computing and Computer Assisted Intervention - MICCAI 2019. MICCAI 2019. Lecture Notes in Computer Science, 2019, vol. 11769, pp. 148-156.
[35] J. Peng and Y. Wang, "Medical image segmentation with limited supervision: A review of deep network models," IEEE, vol. 9, pp. 36827-36851, 2021.
[36] R. Donners, M. Blackledge, N. Tunariu, C. Messiou, E. M. Merkle, and D.-M. Koh, "Quantitative Whole-Body Diffusion - Weighted MR Imaging Quantitative Whole body Diffusion DWI MR Imaging," Magn. Reson. Imaging Clin. N. Am., vol. 26, no. 4, pp. 479-495, 2018.
[37] K. Zormpas-Petridis, N. Tunariu and C. Messiou, "Accelerating whole-body diffusion-weighted mri with deep learning-based denoising image filters," Radiol. Artif. Intell., vol. 3, no. 5, 2021.
[38] N. Tunariu et al., "What's New for Clinical Whole-body MRI (WB-MRI) in the 21st Century," Br J Radiol, vol. 93, no. May, pp. 1-13, 202.
[39] K. Ruchalski et al., "Pretreatment visceral metastases in castration resistant metastatic prostate cancer: role in prediction versus actual site of disease progression," Cancer Imaging, vol. 22, no. 1, pp. 1-9, 202.
[40] L. Pour et al., "Soft-tissue extramedullary multiple myeloma prognosis is significantly worse in comparison to bone-related extramedullary relapse," Haematologica, vol. 99, no. 2, pp. 360-364, 2014.

## Claims

1. A computer-implemented method of delineating one or more parts of a body within a diffusion weighted MRI 3D patient image of a human or animal body, the method including:
providing the diffusion weighted MRI 3D patient image of the human or animal body, the patient image being formed of plural slices stacked along a direction of the body;
analysing the patient image to identify different contiguous anatomical regions of the body, the regions being distributed along said direction such that each slice of the patient image is allocated to a respective region;
providing an atlas of diffusion weighted MRI 3D ground truth images of plural other corresponding bodies containing the regions, each ground truth image being formed of plural slices stacked along a corresponding direction of the respective body, the different regions of the body being pre-identified for each ground truth image such that each slice of that ground truth image is allocated to a respective region, and one or more parts of the body of each ground truth image being pre-delineated;
registering each ground truth image to the patient image by: translating and stretching each ground truth image in its corresponding direction to align the identified regions of that ground truth image with the corresponding identified regions of the patient image;
identifying a transformation of each registered ground truth image that matches the pre-delineated parts of the body of that registered ground truth image to the corresponding parts of the body of the patient image by minimising a cost function; and
segmenting the patient image by obtaining a probability image for the corresponding parts of the body of the patient image, wherein the probability image combines the pre-delineated parts of the bodies of the ground truth images transformed according to their respective non-linear deformable transformations and weighted according to their respective cost-functions.

2. The computer-implemented method according to claim 1, wherein the diffusion weighted MRI 3D patient image of a human or animal body contains a skeleton, the diffusion weighted MRI 3D ground truth images of other corresponding bodies contain skeletons, the patient image is analysed to identify different skeletal regions of the body, and each ground truth image is registered to the patient image by translating and stretching each ground truth image in its corresponding direction to align the identified skeletal regions of that ground truth image with the corresponding identified skeletal regions of the patient image.

3. The computer-implemented method according to claim 1 or 2, wherein the pre-delineated parts of the body include the skeleton and/or one or more soft tissues.

4. The computer-implemented method according to any one of the previous claims, wherein the analysing of the patient image to identify different regions of the body is performed using a neural network; and
preferably wherein the identified different regions of the body include cervical, thoracic, lumbar and pelvic regions.

5. The computer-implemented method according to any one of the previous claims, wherein said direction of the body of the patient image, and the corresponding directions of the ground truth images are craniocaudal directions, the plural slices of the patient image and the plural slices of the ground truth images being axial slices.

6. The computer-implemented method according to any one of the previous claims, further including:
analysing the patient image, for example using a machine learning classifier, to delineate a reference feature, such as a spinal cord, extending in said direction;
wherein the ground truth images are images of plural other corresponding bodies having the reference feature; and
wherein the registering of each ground truth image to the patient image also includes: translating in-plane each slice of each translated and stretched ground truth image to align the reference feature of the ground truth image in that slice with the reference feature of the patient image in a corresponding slice.

7. The computer-implemented method according to claim 6, wherein the aligning of the reference feature of the ground truth image with the reference feature of the patient image in a corresponding slice is performed by matching respective centroids of the reference features.

8. The computer-implemented method according to any one of the previous claims, wherein the identified transformation of each registered ground truth image that matches the pre-delineated parts of the body of that registered ground truth image to the corresponding parts of the body of the patient image is a non-linear deformable transformation.

9. The computer-implemented method according to any one of the previous claims, wherein the cost function is a mean square error cost function.

10. A computer-implemented procedure for identifying possible regions of disease in a human or animal body, the procedure including:
performing the method of any one of the previous claims; and
using the probability image to identify possible regions of disease, such as metastatic bone disease.

11. The computer-implemented procedure according to claim 10, wherein the probability image is used by combining the probability image with other information, such as apparent diffusion coefficient information or high b-value image signal information, derived from the diffusion weighted MRI 3D patient image of the human or animal body.

12. A computer system programmed to perform the method of any one of claims 1 to 9.

13. A computer program comprising code which, when the code is executed on a computer, causes the computer to perform the method of any one of claims 1 to 9.

14. A computer readable medium storing the computer program of claim 13.

15. An imaging system for performing diffusion-weighted MRI, the system including:
a magnetic resonance imaging scanner for acquiring a diffusion-weighted MRI 3D patient image of a human or animal body; and
the computer system according to claim 12, the computer system being configured to communicate with the scanner such that the computer system is provided with the acquired image.

16. A method of training a computer-implemented machine learning model, such as a neural network, which receives an input which is a diffusion weighted MRI 3D patient image of a human or animal body, and produces in response thereto an output which is a probability image corresponding to the diffusion weighted MRI 3D patient image for one or more parts of the body, the method including:
performing the method of any one of claims 1 to 9 for plural diffusion weighted MRI 3D patient images to form a training data set in which each of the diffusion weighted MRI 3D patient images is paired with a corresponding probability image for one or more parts of the body; and
training the machine learning model using the training data set to minimise a cost function that, when each diffusion weighted MRI 3D patient image from the training data set is inputted into the model, measures similarity between the output of the model and the corresponding probability image.

## Patentansprüche

1. Computerimplementiertes Verfahren zum Abgrenzen eines oder mehrerer Körperteile innerhalb eines diffusionsgewichteten MR-3D-Patientenbilds eines menschlichen oder tierischen Körpers, wobei das Verfahren Folgendes umfasst:
Bereitstellen des diffusionsgewichteten MR-3D-Patientenbilds des menschlichen oder tierischen Körpers, wobei das Patientenbild aus mehreren Schnitten gebildet wird, die entlang einer Richtung des Körpers gestapelt sind;
Analysieren des Patientenbilds, um verschiedene durchgängige anatomische Regionen des Körpers zu identifizieren, wobei die Regionen entlang der Richtung verteilt sind, sodass jeder Schnitt des Patientenbilds einer entsprechenden Region zugewiesen wird;
Bereitstellen eines Atlas von diffusionsgewichteten MR-3D-Ground-Truth-Bildern mehrerer anderer entsprechender Körper, die die Regionen enthalten, wobei jedes Ground-Truth-Bild aus mehreren Schnitten gebildet wird, die entlang einer entsprechenden Richtung des entsprechenden Körpers gestapelt sind, wobei die verschiedenen Regionen des Körpers für jedes Ground-Truth-Bild voridentifiziert sind, sodass jeder Schnitt dieses Ground-Truth-Bilds einer entsprechenden Region zugewiesen ist, und wobei ein oder mehrere Körperteile jedes Ground-Truth-Bilds bereits vorab abgegrenzt sind;
Zurdeckungbringen jedes Ground-Truth-Bilds mit dem Patientenbild durch: Translatieren und Ausdehnen jedes Ground-Truth-Bilds in dessen entsprechender Richtung, um die identifizierten Regionen dieses Ground-Truth-Bilds an den entsprechenden identifizierten Regionen des Patientenbilds auszurichten;
Identifizieren einer Transformation jedes zur Deckung gebrachten Ground-Truth-Bilds, die die vorab abgegrenzten Körperteile dieses zur Deckung gebrachten Ground-Truth-Bilds mit den entsprechenden Körperteilen des Patientenbilds abgleicht, indem eine Kostenfunktion minimiert wird; und
Segmentieren des Patientenbilds durch Erhalten eines Wahrscheinlichkeitsbilds für die entsprechenden Körperteile des Patientenbilds, wobei das Wahrscheinlichkeitsbild die vorab abgegrenzten Körperteile der Ground-Truth-Bilder, die gemäß ihren entsprechenden nichtlinearen deformierbaren Transformationen transformiert und gemäß ihren entsprechenden Kostenfunktionen gewichtet wurden, kombiniert.

2. Computerimplementiertes Verfahren nach Anspruch 1, wobei das diffusionsgewichtete MR-3D-Patientenbild eines menschlichen oder tierischen Körpers ein Skelett enthält, wobei die diffusionsgewichteten MR-3D-Ground-Truth-Bilder anderer entsprechender Körper Skelette enthalten, wobei das Patientenbild analysiert wird, um verschiedene Skelettregionen des Körpers zu identifizieren und wobei jedes Ground-Truth-Bild mit dem Patientenbild zur Deckung gebracht wird, indem jedes Ground-Truth-Bild in dessen entsprechende Richtung translatiert und ausgedehnt wird, um die identifizierten Skelettregionen dieses Ground-Truth-Bilds an den entsprechenden identifizierten Skelettregionen des Patientenbilds auszurichten.

3. Computerimplementiertes Verfahren nach Anspruch 1 oder 2, wobei die vorab abgegrenzten Körperteile das Skelett und/oder ein oder mehrere Weichgewebe umfassen.

4. Computerimplementiertes Verfahren nach einem der vorangegangenen Ansprüche, wobei das Analysieren des Patientenbilds zum Identifizieren verschiedener Regionen des Körpers unter Verwendung eines neuronalen Netzes durchgeführt wird; und
wobei die identifizierten verschiedenen Regionen des Körpers vorzugsweise zervikale, thorakale, lumbale und pelvine Regionen umfassen.

5. Computerimplementiertes Verfahren nach einem der vorangegangenen Ansprüche, wobei die Richtung des Körpers des Patientenbilds und die entsprechenden Richtungen der Ground-Truth-Bilder kraniokaudale Richtungen sind, wobei die mehreren Schnitte des Patientenbilds und die mehreren Schnitte der Ground-Truth-Bilder axiale Schnitte sind.

6. Computerimplementiertes Verfahren nach einem der vorangegangenen Ansprüche, das ferner Folgendes umfasst:
Analysieren des Patientenbilds, beispielsweise unter Verwendung eines Klassifikators maschinellen Lernens, um ein Referenzmerkmal, z. B. ein Rückenmark, das sich in die Richtung erstreckt, abzugrenzen;
wobei die Ground-Truth-Bilder Bilder mehrerer anderer entsprechender Körper sind, die das Referenzmerkmal aufweisen; und
wobei das Zurdeckungbringen jedes Ground-Truth-Bilds mit dem Patientenbild ebenfalls Folgendes umfasst: Translatieren jedes Schnitts jedes translatierten und ausgedehnten Ground-Truth-Bilds innerhalb der Ebene, um das Referenzmerkmal des Ground-Truth-Bilds in diesem Schnitt an dem Referenzmerkmal des Patientenbilds in einem entsprechenden Schnitt auszurichten.

7. Computerimplementiertes Verfahren nach Anspruch 6, wobei das Ausrichten des Referenzmerkmals des Ground-Truth-Bilds mit dem Referenzmerkmal des Patientenbilds in einem entsprechenden Schnitt durch Abgleichen entsprechender Zentroide der Referenzmerkmale durchgeführt wird.

8. Computerimplementiertes Verfahren nach einem der vorangegangenen Ansprüche, wobei die identifizierte Transformation jedes zur Deckung gebrachten Ground-Truth-Bilds, die die vorab abgegrenzten Körperteilen dieses zur Deckung gebrachten Ground-Truth-Bilds mit den entsprechenden Körperteilen des Patientenbilds abgleicht, eine nichtlineare deformierbare Transformation ist.

9. Computerimplementiertes Verfahren nach einem der vorangegangenen Ansprüche, wobei die Kostenfunktion eine mittlere quadratische Fehlerkostenfunktion ist.

10. Computerimplementierter Ablauf zum Identifizieren möglicher Krankheitsregionen in einem menschlichen oder tierischen Körper, wobei der Ablauf Folgendes umfasst:
Durchführen eines Verfahrens nach einem der vorangegangenen Ansprüche; und
Verwenden des Wahrscheinlichkeitsbilds, um mögliche Krankheitsregionen, z. B. metastatische Knochenerkrankungen, zu identifizieren.

11. Computerimplementierter Ablauf nach Anspruch 10, wobei das Wahrscheinlichkeitsbild durch Kombinieren des Wahrscheinlichkeitsbilds mit anderen Informationen, wie z. B. Informationen über den scheinbaren Diffusionskoeffizienten oder Bildsignalinformationen mit hohem b-Wert, abgeleitet von dem diffusionsgewichteten MR-3D-Patientenbild des menschlichen oder tierischen Körpers, verwendet wird.

12. Computersystem, programmiert zum Durchführen eines Verfahrens nach einem der Ansprüche 1 bis 9.

13. Computerprogramm, umfassend Code, der, wenn der Code auf einem Computer ausgeführt wird, bewirkt, dass der Computer ein Verfahren nach einem der Ansprüche 1 bis 9 durchführt.

14. Computerlesbares Medium, das ein Computerprogramm nach Anspruch 13 speichert.

15. Bildgebungssystem zum Durchführen von diffusionsgewichteter MR, wobei das System Folgendes umfasst:
einen Magnetresonanz-Bildgebungsscanner zum Erfassen eines diffusionsgewichteten MR-3D-Patientenbilds eines menschlichen oder tierischen Körpers; und
ein Computersystem nach Anspruch 12, wobei das Computersystem ausgelegt ist, um mit dem Scanner zu kommunizieren, sodass das erfasste Bild an dem Computersystem bereitgestellt wird.

16. Verfahren zum Trainieren eines computerimplementierten Modells maschinellen Lernens, wie z. B. ein neuronales Netzwerk, das eine Eingabe empfängt, die ein diffusionsgewichtetes MR-3D-Patientenbild eines menschlichen oder tierischen Körpers ist, und als Reaktion darauf eine Ausgabe erzeugt, die ein Wahrscheinlichkeitsbild ist, das dem diffusionsgewichteten MR-3D-Patientenbild für einen oder mehrere Körperteile entspricht, wobei das Verfahren Folgendes umfasst:
Durchführen eines Verfahrens nach einem der Ansprüche 1 bis 9 für mehrere diffusionsgewichtete MR-3D-Patientenbilder, um einen Trainingsdatensatz zu bilden, in dem jedes der diffusionsgewichteten MR-3D-Patientenbilder mit einem entsprechenden Wahrscheinlichkeitsbild für einen oder mehrere Körperteile gepaart wird; und
Trainieren des Modells maschinellen Lernens unter Verwendung des Trainingsdatensatzes, um eine Kostenfunktion zu minimieren, die, wenn jedes diffusionsgewichtete MR-3D-Patientenbild aus dem Trainingsdatensatz in das Modell eingegeben wird, eine Ähnlichkeit zwischen der Ausgabe des Modells und dem entsprechenden Wahrscheinlichkeitsbild misst.

## Revendications

1. Procédé mis en œuvre par ordinateur permettant de délimiter une ou plusieurs parties d'un corps dans une image de patient 3D d'IRM de diffusion d'un corps humain ou animal, le procédé incluant les étapes consistant à :
fournir l'image de patient 3D d'IRM de diffusion du corps humain ou animal, l'image de patient étant formée de plusieurs tranches empilées le long d'une direction du corps ;
analyser l'image de patient pour identifier différentes régions anatomiques contiguës du corps, les régions étant réparties le long de ladite direction de telle sorte que chaque tranche de l'image de patient soit attribuée à une région respective ;
fournir un atlas d'images de réalité de terrain 3D d'IRM de diffusion de plusieurs autres corps correspondants contenant les régions, chaque image de réalité de terrain étant formée de plusieurs tranches empilées le long d'une direction correspondante du corps respectif, les régions différentes du corps étant pré-identifiées pour chaque image de réalité de terrain de telle sorte que chaque tranche de cette image de réalité de terrain soit attribuée à une région respective, et une ou plusieurs parties du corps de chaque image de réalité de terrain étant pré-délimitées ;
enregistrer chaque image de réalité de terrain sur l'image de patient par : translation et étirement de chaque image de réalité de terrain dans sa direction correspondante pour aligner les régions identifiées de cette image de réalité de terrain avec les régions identifiées correspondantes de l'image de patient ;
identifier une transformation de chaque image de réalité de terrain enregistrée qui vient en correspondance avec les parties pré-délimitées du corps de cette image de réalité de terrain enregistrée pour les parties correspondantes du corps de l'image de patient en minimisant une fonction de coût ; et
segmenter l'image de patient en obtenant une image de probabilité pour les parties correspondantes du corps de l'image de patient, dans lequel l'image de probabilité combine les parties pré-délimitées des corps des images de réalité de terrain transformées en fonction de leurs transformations déformables non linéaires respectives et pondérées conformément à leurs fonctions de coût respectives.

2. Procédé mis en œuvre par ordinateur selon la revendication 1, dans lequel l'image de patient 3D d'IRM de diffusion d'un corps humain ou animal contient un squelette, les images de réalité de terrain 3D d'IRM de diffusion d'autres corps correspondants contiennent des squelettes, l'image de patient est analysée pour identifier différentes régions de squelette du corps, et chaque image de réalité de terrain est enregistrée sur l'image de patient en translatant et en étirant chaque image de réalité de terrain dans sa direction correspondante pour aligner les régions de squelette identifiées de cette image de réalité de terrain avec les régions de squelette identifiées correspondantes de l'image de patient.

3. Procédé mis en œuvre par ordinateur selon la revendication 1 ou 2, dans lequel les parties pré-délimitées du corps incluent le squelette et/ou un ou plusieurs tissus mous.

4. Procédé mis en œuvre par ordinateur selon l'une quelconque des revendications précédentes, dans lequel l'analyse de l'image de patient pour identifier différentes régions du corps est effectuée en utilisant un réseau neuronal ; et
de préférence dans lequel les différentes régions identifiées du corps incluent des régions cervicale, thoracique, lombaire et pelvienne.

5. Procédé mis en œuvre par ordinateur selon l'une quelconque des revendications précédentes, dans lequel ladite direction du corps de l'image de patient et les directions correspondantes des images de réalité de terrain sont des directions cranio-caudales, les plusieurs tranches de l'image de patient et les plusieurs tranches des images de réalité de terrain étant des tranches axiales.

6. Procédé mis en œuvre par ordinateur selon l'une quelconque des revendications précédentes, incluant en outre l'étape consistant à :
analyser l'image de patient, par exemple en utilisant un classificateur d'apprentissage automatique, pour délimiter une caractéristique de référence, telle qu'une moelle épinière, s'étendant dans ladite direction ;
dans lequel les images de réalité de terrain sont des images de plusieurs autres corps correspondants présentant la caractéristique de référence ; et
dans lequel l'enregistrement de chaque image de réalité de terrain sur l'image de patient comprend également : une translation dans le plan de chaque tranche de chaque image de réalité de terrain translatée et étirée pour aligner la caractéristique de référence de l'image de réalité de terrain dans cette tranche avec la caractéristique de référence de l'image de patient dans une tranche correspondante.

7. Procédé mis en œuvre par ordinateur selon la revendication 6, dans lequel l'alignement de la caractéristique de référence de l'image de réalité de terrain avec la caractéristique de référence de l'image de patient dans une tranche correspondante est effectué en faisant correspondre des centroïdes respectifs des caractéristiques de référence.

8. Procédé mis en œuvre par ordinateur selon l'une quelconque des revendications précédentes, dans lequel la transformation identifiée de chaque image de réalité de terrain enregistrée, qui met en correspondance les parties pré-délimitées du corps de cette image de réalité de terrain enregistrée et les parties correspondantes du corps de l'image de patient, est une transformation déformable non linéaire.

9. Procédé mis en œuvre par ordinateur selon l'une quelconque des revendications précédentes, dans lequel la fonction de coût est une fonction de coût d'erreur quadratique moyenne.

10. Procédure mise en œuvre par ordinateur pour identifier des régions possibles de maladie dans un corps humain ou animal, la procédure incluant les étapes consistant à :
mettre en œuvre le procédé selon l'une quelconque des revendications précédentes ; et
utiliser l'image de probabilité pour identifier de possibles régions de maladie, comme une maladie osseuse métastatique.

11. Procédure mise en œuvre par ordinateur selon la revendication 10, dans laquelle l'image de probabilité est utilisée en combinant l'image de probabilité avec d'autres informations, telles que des informations de coefficient de diffusion apparente ou des informations de signal d'image de valeur b élevée, dérivées de l'image de patient 3D d'IRM de diffusion du corps humain ou animal.

12. Système informatique programmé pour exécuter le procédé selon l'une quelconque des revendications 1 à 9.

13. Programme informatique comprenant un code qui, lorsque le code est exécuté sur un ordinateur, amène l'ordinateur à exécuter le procédé selon l'une quelconque des revendications 1 à 9.

14. Support lisible par ordinateur stockant le programme informatique de la revendication 13.

15. Système d'imagerie pour effectuer une IRM de diffusion, le système incluant :
un scanner d'imagerie par résonance magnétique pour acquérir une image de patient 3D d'IRM de diffusion d'un corps humain ou animal ; et
le système informatique selon la revendication 12, le système informatique étant configuré pour communiquer avec le scanner de telle sorte que le système informatique soit pourvu de l'image acquise.

16. Procédé d'entraînement d'un modèle d'apprentissage automatique mis en œuvre par ordinateur, tel qu'un réseau neuronal, qui reçoit une entrée qui est une image de patient 3D d'IRM de diffusion d'un corps humain ou animal, et produit en réponse à celle-ci une sortie qui est une image de probabilité correspondant à l'image de patient 3D d'IRM de diffusion pour une ou plusieurs parties du corps, le procédé incluant les étapes consistant à :
exécuter le procédé selon l'une quelconque des revendications 1 à 9 pour plusieurs images de patient 3D d'IRM de diffusion afin de former un ensemble de données d'entraînement dans lequel chacune des images de patient 3D d'IRM de diffusion est appariée à une image de probabilité correspondante pour une ou plusieurs parties du corps ; et
entraîner le modèle d'apprentissage machine en utilisant l'ensemble de données d'entraînement pour minimiser une fonction de coût qui, lorsque chaque image de patient 3D d'IRM de diffusion provenant de l'ensemble de données d'entraînement est entrée dans le modèle, mesure la similitude entre la sortie du modèle et l'image de probabilité correspondante.
